(19) Europäisches Patentamt
European Patent Office
Office européen des brevets

(11) EP 4 549 579 A1

(12) **EUROPEAN PATENT APPLICATION**
published in accordance with Art. 153(4) EPC

(43) Date of publication:
**07.05.2025 Bulletin 2025/19**

(21) Application number: **23831626.9**

(22) Date of filing: **30.06.2023**

(51) International Patent Classification (IPC):
***C12Q 1/68*** *(2018.01)* ***A61K 31/4152*** *(2006.01)*
***A61P 21/00*** *(2006.01)* ***A61P 25/00*** *(2006.01)*
***A61P 25/28*** *(2006.01)* ***C12Q 1/686*** *(2018.01)*

(52) Cooperative Patent Classification (CPC):
**A61K 31/4152; A61P 21/00; A61P 25/00; A61P 25/28; C12Q 1/68; C12Q 1/686**

(86) International application number:
**PCT/JP2023/024373**

(87) International publication number:
**WO 2024/005187 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.07.2022 JP 2022106787**

(71) Applicant: **Mitsubishi Tanabe Pharma Corporation**
**Osaka-shi,**
**Osaka 541-8505 (JP)**

(72) Inventors:
- **KUSUNOKI, Aki**
  **Osaka-shi, Osaka 541-8505 (JP)**
- **OKADA, Kinya**
  **Osaka-shi, Osaka 541-8505 (JP)**
- **MURATANI MIKURIYA, Satsuki**
  **Cambridge, Massachusetts 02139 (US)**
- **TAMURA, Makoto**
  **Cambridge, Massachusetts 02139 (US)**

(74) Representative: **J A Kemp LLP**
**80 Turnmill Street**
**London EC1M 5QU (GB)**

(54) **COMPOSITION AND METHOD FOR EVALUATING RESPONSIVENESS OF EDARAVONE**

(57) A composition of the present disclosure contains edaravone and is used for causing a change in expression level of a gene product in a target. The gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2. The composition is also suitably used as a pharmaceutical. The composition is also suitably used for treating or preventing a neurodegenerative disease. The neurodegenerative disease is suitably amyotrophic lateral sclerosis (ALS). The present disclosure also provides a method for evaluating responsiveness of edaravone based on an expression level of the gene product or a change in the expression level.

EP 4 549 579 A1

## Description

[Technical Field]

The present application claims priority based on Japanese Patent Application No. 2022-106787, filed on July 1, 2022, the entire content of which is incorporated by reference into this specification.

The present invention relates to a composition, and a method for evaluating responsiveness of edaravone.

[Technical Background]

Edaravone is an organic compound known as 5-methyl-2-phenyl-2,4-dihydro-3H-pyrazol-3-one (CAS number 89-25-8). Edaravone is used for medical purposes as a brain protective agent or as a therapeutic agent for amyotrophic lateral sclerosis (ALS), a type of neurodegenerative disease (Patent Documents 1 and 2).

ALS is an intractable disease that leads to respiratory failure from initial symptoms such as weakness in hands, movement disorders with fingers and fascicular contraction in upper limbs, through symptoms such as amyotrophia and/or muscular weakness, bulbar paralysis and fascicular contraction in muscles. Neurodegenerative diseases such as ALS have been reported to involve, for example, TDP-43 (TAR DNA-binding protein of 43kDa) protein. TDP-43 is a protein identified as one of components found in the brain of a patient with frontotemporal lobar degeneration (FTLD) or ALS (see Non-Patent Document 1). Onset and progression of a neurodegenerative disease such as ALS are considered to be partly due to formation of aggregates of the TDP-43 protein.

[Related Art]

[Patent Documents]

[Patent Document 1] Japanese Patent Publication No. H5-31523.
[Patent Document 2] U.S. Patent No. 6,933,310.

[Non-Patent Documents]

[Non-Patent Document 1] Arai et al., Biochem Biophys Res Commun, 351 (3): 602-11 (2006).

[Summary of the Invention]

The present inventor has found that exposure of a target to edaravone causes a change in expression level of a gene product of a specific gene. Further, the inventor has also found that the change in expression level can be a useful indicator, such as a biomarker, in exploring responsiveness to edaravone or possibility of having an applicable disease, or the like.

The present disclosure includes an invention related to a composition.

In one embodiment, the composition is used for causing a change in expression level of a gene product in a target.

In one embodiment, the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1, and FAIM2.

Further, the present disclosure also includes an invention related to a method for evaluating responsiveness to edaravone.

In one embodiment, the evaluation method includes a step of evaluating whether or not there is a possibility that a target has responsiveness to edaravone based on a change in expression level of a gene product due to exposure of the target to edaravone.

In one embodiment, the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1, and FAIM2.

Other embodiments will become apparent from descriptions of the present specification and the claims.

According to the present invention, by exposing a target to edaravone, an expression level of a specific gene product can be changed. As a result, an advantageous effect corresponding to a function of a gene can be achieved, and can be used, for example, in treatment or prevention of a disease.

Further, according to the present invention, an expression change of a specific gene product can be used in screening a substance capable of treating or preventing a disease.

Further, according to the present invention, an expression change of a specific gene product can be used in

various assessments and predictions of the presence or absence of a disease and progression of the disease, treatment responsiveness, and the like.

[Brief Description of the Drawings]

[Fig. 1] Fig. 1 shows fluorescence microscope images showing cell morphology of neurons expressing wild-type and mutant TDP-43 proteins when exposed to ethacrynic acid (EA). The images in the lower row of Fig. 1 are enlarged views of the images in the upper row of Fig. 1.

[Fig. 2] Fig. 2 shows fluorescence microscope images showing cell morphology of neurons expressing wild-type and mutant TDP-43 proteins when exposed to ethacrynic acid and edaravone. The images in the lower row of Fig. 2 are enlarged views of the images in the upper row of Fig. 2.

[Fig. 3] Fig. 3 is a graph showing cell survival rates of neurons expressing wild-type and mutant TDP-43 proteins, depending on the presence or absence of exposure to ethacrynic acid and/or edaravone.

[Fig. 4] Fig. 4 is a diagram showing an overview of treatments for experimental groups used in gene product expression analysis.

[Fig. 5] Fig. 5 is a diagram showing an overview of procedures for gene product expression analysis.

[Fig. 6] Fig. 6 is a graph showing expression distribution of the experimental groups.

[Fig. 7] Figs. 7(a) - 7(d) are graphs showing expression changes of gene products depending on the presence or absence of edaravone and/or a pathological condition.

[Fig. 8] Figs. 8(a) - 8(d) are graphs showing expression changes of other gene products depending on the presence or absence of edaravone and/or a pathological condition.

[Fig. 9] Figs. 9(a) - 9(d) are graphs showing expression changes of yet other gene products depending on the presence or absence of edaravone and/or a pathological condition.

[Fig. 10] Figs. 10(a) and 10(b) are graphs showing expression changes of yet other gene products depending on the presence or absence of edaravone and/or a pathological condition.

[Fig. 11] Fig. 11 is a graph showing expression changes of yet other gene products depending on the presence or absence of edaravone and/or a pathological condition.

[Fig. 12] Fig. 12 is a graph showing cell survival rates of neurons expressing wild-type and mutant TDP-43 proteins, and neurons not expressing TDP-43 proteins, depending on the presence or absence of exposure to edaravone.

[Fig. 13] Fig. 13 is a graph showing change rates in neurite length depending on the presence or absence of exposure to edaravone in neurons differentiated from iPS cells induced from cells derived from an ALS patient and a healthy individual.

[Fig. 14] Fig. 14 is a graph showing change rates in the number of death cells depending on the presence or absence of exposure to edaravone in neurons differentiated from iPS cells induced from cells derived from an ALS patient and a healthy individual.

[Fig. 15] Fig. 15 shows fluorescence microscope images showing the intracellular localization of TDP-43 when exposed to edaravone in neurons differentiated from iPS cells induced from cells derived from an ALS patient and a healthy individual.

[Mode for Carrying Out the Invention]

In the following, the present invention is described based on preferred embodiments thereof.

The present disclosure includes a composition containing edaravone. In one embodiment, the composition may be used as a pharmaceutical, or it may be used as a non-pharmaceutical, such as a reagent, for example.

In either case, by causing a change in expression level of a gene product of a specific gene in a target, an advantageous effect corresponding to a type or a degree of expression of the gene and its gene product can be obtained.

Details of the gene and its gene product will be described later.

In the present specification, "edaravone" includes 5-methyl-2-phenyl-2,4-dihydro-3H-pyrazol-3-one itself and its tautomers, derivatives thereof, and salts, hydrates, or solvates thereof.

In one embodiment, edaravone preferably includes at least one selected from 5-methyl-2-phenyl-2,4-dihydro-3H-pyrazol-3-one and its tautomers,and salts, hydrates, or solvates thereof. That is, edaravone preferably includes at least one selected from isomers represented by the following structural formula (1) and structural formula (2), and salts, hydrates, or solvates thereof.

Edaravone can be produced, for example, using a production method described in European Patent Publication No. 208874.

[Chemical Formula 1]

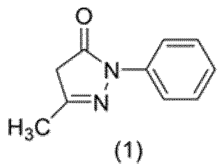
(1)

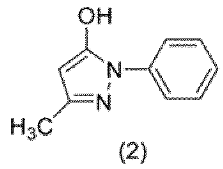
(2)

In the present specification, unless otherwise specified, the term "gene product" is used to include transcription products, reverse transcription products, and translation products derived from a gene, and can be one or more of these products.

A gene product may be one or more transcription products only, one or more reverse transcription products only, one or more translation products only, or a combination of one or more transcription products and one or more translation products.

Examples of transcription products include various types of RNA, such as mRNA and noncoding RNA. A transcription product is preferably mRNA, whether mature or not, and more preferably mature mRNA.

An example of a reverse transcription product is cDNA.

An example of a translation product is a protein produced through transcription and translation. A translation product is preferably a protein, regardless of the presence or absence of post-translational modification.

In one embodiment, the composition containing edaravone is preferably used as a pharmaceutical. That is, in one embodiment, the composition is a pharmaceutical composition containing edaravone as an active ingredient.

Bsing the above-described composition as a pharmaceutical, it is possible to change an expression level of a specific gene product in a target, for example, to maintain a brain function at a normal level or improve the brain function, or to contribute to treatment or prevention of diseases such as neurodegenerative diseases, muscle diseases, vascular disorders, and various inflammatory diseases.

In the present specification, the term "target" is not particularly limited as long as an expression level of a gene product can be measured. Examples of "target" include humans and non-human animals, as well as samples derived from these animals, regardless of the presence or absence of a disease or environments such as in vivo, ex vivo, or in vitro.

The humans and non-human animals are preferably mammals. That is, a target of exposure to the composition is preferably a mammal or a sample derived from a mammal.

Examples of non-human animals include rodents such as rats, mice, and guinea pigs; and non-human mammals such as monkeys, pigs, dogs, and cats.

Examples of samples include, but are not limited to, one or more of tissues, cells, and body fluids.

Examples of tissues include brain (such as cerebrum and cerebellum), spinal cord, stomach, pancreas, kidneys, liver, adrenal glands, skin, muscles (such as skeletal and smooth muscles), lungs, intestines (such as large and small intestines), heart, blood vessels, and the like.

Examples of cells include differentiated cells, progenitor cells, or stem cells that form tissues. Taking brain-derived cells as an example, examples of brain-derived samples include neuronal cells (neurons), glial cells, neural-related differentiated cells such as astrocytes, neural stem cells, and the like.

Examples of body fluids include liquid components such as cerebrospinal fluid, blood, serum, plasma, saliva, urine, and sweat, or extracts thereof.

These samples can typically be collected from living or dead animals using commonly known methods.

As other forms of cells, various cultured cells can be used, such as primary cultured cells, immortalized cell lines,

or pluripotent stem cells such as ES cells and iPS cells. Among these, iPS cells may be induced from cells derived from an ALS patient or cells with an ALS risk mutation as a heterozygous mutation in the TARDBP gene. Cells differentiated from the pluripotent stem cells described above, for example, neurons, are also included in the target in the present disclosure.

In one embodiment, the above-described composition is suitably used for treating or preventing one or more of neurodegenerative diseases, muscle diseases, vascular disorders, and inflammatory diseases.

Examples of neurodegenerative diseases include neurodegenerative diseases with motor function impairment and neurodegenerative diseases with cognitive function impairment.

Examples of neurodegenerative diseases with motor function impairment include amyotrophic lateral sclerosis (ALS), spinal muscular atrophy (SMA), progressive bulbar palsy, primary lateral sclerosis (PLS), arthrogryposis multiplex congenita (AMC), and the like.

Examples of neurodegenerative diseases with cognitive function impairment include Alzheimer's disease, frontotemporal lobar degeneration (FTD), and the like.

Examples of muscle diseases include muscular dystrophy, and the like.

Examples of vascular disorders include cerebral infarction, cerebral hemorrhage, and the like.

Examples of inflammatory diseases include systemic inflammatory diseases such as multiple sclerosis and systemic sclerosis, and localized inflammatory diseases such as stomatitis

Among these diseases, the above-described composition as a pharmaceutical composition is used preferably for treating or preventing neurodegenerative diseases, more preferably for treating or preventing neurodegenerative diseases accompanied by motor dysfunction, and even more preferably for treating or preventing amyotrophic lateral sclerosis (ALS).

In the present specification, "treatment" includes suppression of progression, improvement, alleviation, complete cure, and the like of a disease.

In the specification, "prevention" includes prevention of onset of a disease, prevention of recurrence of a disease, and the like.

In one embodiment, the above-described composition is also preferably used for treatment or prevention of a disease accompanied by the presence of TDP-43 mutant protein or abnormal intracellular localization of TDP-43. Examples of such diseases include, but are not limited to, amyotrophic lateral sclerosis (ALS) and the like. Examples of TDP-43 mutant protein include, but are not limited to, C-terminal fragments of wild-type TDP-43 protein (for example, amino acid residues 208 - 414 from the N-terminus of wild-type TDP-43 protein) and point mutations of wild-type TDP-43 protein (for example, G294A, G298S, A315T, and Q343R). Examples of wild-type TDP-43 protein include an amino acid sequence described in Accession number:
NP_031401.1.

Regardless of a disease, by using the above-described composition, an expression level of a specific gene product can be changed by exposure to edaravone. By the expression level of the gene product or the change in the expression level, cellular dysfunction or cellular damage such as cell death can be effectively suppressed. As a result, the disease can be treated or prevented, or symptoms of the disease can be alleviated. As shown in examples to be described later, the above-described composition is advantageous in that it is capable of treating or preventing diseases that potentially involve TDP-43 mutant protein, such as ALS. When TDP-43 mutant protein is present, it is presumed that TDP-43 protein aggregates form within cells, making cellular damage more likely to occur. Therefore, by using the above-described composition, cellular damage can be effectively suppressed.

The edaravone-containing composition of the present disclosure is used for causing a change in expression level of a specific gene product.

As a result of diligent studies regarding a mechanism of action of edaravone, the present inventor has found that, as shown in the examples to be described later, expression levels of gene products of specific genes change depending on the presence or absence of exposure to edaravone. Further, the inventor also has found that the expression levels of these gene products also can change in specific diseases. Further, the inventor also has found that an expression change in the presence of edaravone can contribute to suppression of cellular damage, and consequently to prediction, or treatment or prevention of a disease caused by cellular damage. That is, through the studies, the present inventor has found the above-described composition has unknown attributes and new uses, and thus has accomplished the present invention.

In the present specification, the term "change" means that an amount of a substance is increased or decreased compared to a measured value or reference value of a comparison target, and also includes, in addition to a magnitude of the amount or a ratio, appearance of the amount from an undetectable state to a detectable state, and disappearance of the amount from a detectable state to an undetectable state.

Whether or not an expression level has changed is determined as follows: when a measured value of an expression level of a gene product in a target of determination is larger or smaller than a measured value or reference value of the expression level of the gene product in a comparison target, it is determined that the expression level has changed. The change in expression level may be determined based on a magnitude of a numerical number of the measured value itself, or based on a magnitude of a ratio calculated from the measured value. Further, the change in expression may be

determined based on a magnitude of an arithmetic mean value or a median value calculated from multiple measured values or ratios, or it may be determined that it has changed based on the presence of a statistically significant difference.

The change in expression level is evaluated by comparing expression levels of measurable molecules derived from the same gene between two experimental groups as comparison targets, on a condition that the two experimental groups are of the same animal species or derived from the same animal species.

Examples of molecules for which expression levels can be measured include, but are not limited to, RNA, which is a transcription product, cDNA, which is a reverse transcription product of the RNA, proteins, which are translation products, and the like.

When evaluating the change in expression level using a ratio of measured values, it may be determined that the expression level has changed when a ratio (R2/R1) of a measured value R2 of an expression level of a gene product in an experimental group as a target of determination to a measured value or reference value R1 as a reference is, for example, 1.01 times or more, for example, 1.10 times or more, for example, 1.30 times or more, for example, 1.50 times or more, for example, 1.70 times or more, or for example, 2.0 times or more (log2 ratio of 1 or more). In this case, it is also possible to determine that the experimental group as a target of determination has preferably changed such that the expression level is increased.

Further, it may be determined that the expression level has changed when the R2/R1 ratio is, for example, 0.99 times or less, for example, 0.90 times or less, for example, 0.80 times or less, for example, 0.70 times or less, for example, 0.60 times or less, or for example, 0.50 times or less (log2 ratio of -1 or less). In this case, it is also possible to determine that the experimental group as a target of determination has preferably changed such that the expression level is decreased.

The composition of the present disclosure is suitably used for causing a change in expression level of a transcription product of, or a translation product encoded by, one or more genes selected from a group of specific genes discovered through diligent studies of the present inventor. The transcription product and translation product of which an expression level is changed may be one of these, or two or more of these.

In the following, names of genes of which expression levels of gene products change due to the presence of edaravone and/or a disease are exemplified, but are not limited to these. For convenience of description, the following genes are denoted as human genes unless otherwise specified. However, the present disclosure also includes orthologs of animal species other than humans. Base sequences or protein amino acid sequences of these genes can be searched for using a public database or the like.

In the present specification, gene groups of which expression levels of gene products change due to the presence of edaravone and/or a disease (the following (A) and (B)) are collectively referred to as "specific genes." Description regarding specific genes applies to all embodiments described in the present specification unless otherwise specified.

<Specific Genes>

Examples of genes of which expression levels of gene products increase due to the presence of a disease and the absence of edaravone, and decrease due to the presence of both a disease and edaravone, are shown below as (A).

In one embodiment, the above-described composition is used for decreasing an expression level of a gene product in a target with a disease, for any single gene, any combination of two or more genes, or a combination of all genes from the gene group (A).

In one embodiment, the above-described disease is preferably a neurodegenerative disease.

[Gene Group (A)]

- KAZALD1 (Kazal Type Serine Peptidase Inhibitor Domain 1)
- SBK1 (SH3-binding domain kinase 1)
- SCN2A (sodium voltage-gated channel alpha subunit 2)
- UBE2L6 (ubiquitin conjugating enzyme E2 L6)
- ALPL (alkaline phosphatase)
- NTM (neurotrimin)
- PTTG1 (regulator of sister chromatid separation; Securin)

- ITGB4 (integrin subunit beta 4)
- HAUS4 (HAUS augmin like complex subunit 4)
- DCTD (dCMP deaminase)
- MT2A (metallothionein 2A)
- ASF1B (anti-silencing function 1B histone chaperone)
- FCSK (fucose kinase)
- MAST1 (microtubule associated serine/threonine kinase 1)

Further, examples of genes of which expression levels of gene products decrease due to the presence of a disease and the absence of edaravone, and increase due to the presence of both a disease and edaravone, are shown below as (B).

In one embodiment, the above-described composition is used for increasing an expression level of a gene product in a target with a disease, for the gene group (B).

In one embodiment, the above-described disease is preferably a neurodegenerative disease.

[Gene Group (B)]

- FAIM2 (Fas apoptotic inhibitory molecule 2)

KAZALD1 is a gene that encodes a type of protein belonging to an insulin growth factor (IGF) binding protein superfamily. An expression change of KAZALD1 can contribute to cell proliferation or a change in function or expression level of IGF. It has been found that an expression level of a gene product of this gene increases in the presence of TDP-43 mutant protein, which is a pathological condition of a neurodegenerative disease, and further, decreases in the presence of edaravone.

There is a possibility that an expression change of IGF is associated with a risk change of a neurodegenerative disease such as ALS through some mechanism. Therefore, it is considered that decreasing the expression level of KAZALD1 is useful in decreasing the risk of developing or progressing a neurodegenerative disease through regulation of IGF activity, and enhancing the possibility of treatment or prognosis prediction of a neurodegenerative disease such as ALS.

SBK1 is a gene that encodes a type of serine/threonine protein kinase. An expression change of SBK1 can contribute to cancer progression or cell proliferation. It has been found that an expression level of a gene product of this gene increases in the presence of TDP-43 mutant protein, which is a pathological condition of a neurodegenerative disease, and further, decreases in the presence of edaravone.

It is considered that decreasing the expression level of SBK1 is useful in suppressing the occurrence of cellular damage by suppressing the formation of TDP-43 protein aggregates through phosphorylation, or enhancing the possibility of treatment or prognosis prediction for a neurodegenerative disease such as ALS.

SCN2A is a gene that encodes a type of protein belonging to a voltage-gated sodium channel family in excitatory neurons. An expression change of SCN2A can contribute to occurrence of epilepsy or a developmental disorder. Further, during neuronal cellular damage, the function of SCN2A can become excessively activated, potentially worsening the cellular damage. It has been found that an expression level of a gene product of this gene increases in the presence of TDP-43 mutant protein, which is a pathological condition of a neurodegenerative disease, and further, decreases in the presence of edaravone.

By decreasing the expression level of SCN2A, the occurrence of cellular damage can be suppressed. Further, it is considered that it is useful in that, along with the decrease in the expression level of SCN2A, a possibility of performing treatment or prognosis prediction of a neurodegenerative disease such as ALS can be enhanced. In particular, it is considered that decreasing the expression level of SCN2A is advantageous in that the function of SCN2A can be suppressed during occurrence of neuronal cellular damage and as a result, progression of the cellular damage can be effectively suppressed.

UBE2L6 is a gene that encodes a type of protein belonging to an ubiquitin-conjugating enzyme family. It is considered that an expression change of UBE2L6 contributes to protein degradation and suppresses autophagy in a

pathological condition such as cancer. It has been found that an expression level of a gene product of this gene increases in the presence of TDP-43 mutant protein, which is a pathological condition of a neurodegenerative disease, and further, decreases in the presence of edaravone.

When autophagy is suppressed, degradation of abnormal proteins such as TDP-43 protein aggregates may be hindered, potentially leading to progression of the pathological condition. Therefore, by decreasing the expression level of UBE2L6, autophagy can be promoted and degradation of abnormal proteins can be promoted. As a result, it is considered that it is useful in suppressing the occurrence of cellular damage, or enhancing the possibility of treatment or prognosis prediction for a neurodegenerative disease such as ALS.

ALPL is a gene that encodes a type of protein belonging to an alkaline phosphatase family.

ALPL is highly expressed in axons or dendrites in neurons. It has been found that an expression level of a gene product of this gene increases in the presence of TDP-43 mutant protein, which is a pathological condition of a neurodegenerative disease, and further, decreases in the presence of edaravone.

An increase in expression level of ALPL can contribute to occurrence or progression of a neurodegenerative disease such as ALS through some mechanism in neuronal cells. Therefore, it is considered that decreasing the expression level of ALPL is useful in suppressing the occurrence of cellular damage or enhancing the possibility of treatment or prognosis prediction for a neurodegenerative disease such as ALS.

NTM is a gene that encodes a type of protein belonging to an IgLON family of cell adhesion molecules. An expression change of NTM can contribute to elongation of neurites and axons, or cell-to-cell adhesion. It has been found that an expression level of a gene product of this gene increases in the presence of TDP-43 mutant protein, which is a pathological condition of a neurodegenerative disease, and further, decreases in the presence of edaravone. In an autoimmune neurological disease involving antibodies against the IgLON family, phosphorylated tau protein may accumulate in the thalamus and brainstem tegmentum, potentially leading to symptoms similar to those of a bulbar palsy-type motor neuron disease. Therefore, it is considered that decreasing the expression level of NTM is useful in reducing accumulation of abnormal proteins or adverse effects associated with the accumulation, thereby enhancing the possibility of treatment or prognosis prediction of various neurodegenerative diseases such as bulbar palsy-type ALS.

PTTG1 is a gene that encodes securin, which is a protein that contributes to cellular mitosis. An expression change of PTTG1 can contribute to regulation of cell division or tumor formation. It has been found that an expression level of a gene product of this gene increases in the presence of TDP-43 mutant protein, which is a pathological condition of a neurodegenerative disease, and further, decreases in the presence of edaravone.

An increase in expression level of PTTG1 can contribute to occurrence or progression of a neurodegenerative disease such as ALS through some mechanism related to cell division. Therefore, it is considered that decreasing the expression level of PTTG1 is useful in suppressing the occurrence of cellular damage or enhancing the possibility of treatment or prognosis prediction for a neurodegenerative disease such as ALS.

ITGB4 is a gene that encodes a type of protein belonging to an integrin family. An expression change of ITGB4 can contribute to cell proliferation, adhesion, and migration, or tissue formation and repair. It has been found that an expression level of a gene product of this gene increases in the presence of TDP-43 mutant protein, which is a pathological condition of a neurodegenerative disease, and further, decreases in the presence of edaravone.

An increase in expression level of ITGB4 can contribute to occurrence or progression of a neurodegenerative disease such as ALS through some mechanism related to cell proliferation or repair. Therefore, it is considered that decreasing the expression level of ITGB4 is useful in suppressing the occurrence of cellular damage or enhancing the possibility of treatment or prognosis prediction for a neurodegenerative disease such as ALS.

HAUS4 is a gene that encodes a type of protein that contributes to microtubule formation during cellular mitosis. An expression change of HAUS4 can contribute to regulation of cell division or elongation of axons and dendrites. It has been found that an expression level of a gene product of this gene increases in the presence of TDP-43 mutant protein, which is a pathological condition of a neurodegenerative disease, and further, decreases in the presence of edaravone.

An increase in expression level of HAUS4 has been confirmed in an ALS disease animal model and can contribute to occurrence or progression of a neurodegenerative disease such as ALS. Therefore, it is considered that decreasing the expression level of HAUS4 is useful in contributing to the formation or functional maintenance of normal neuronal cells, suppressing the occurrence of cellular damage, or enhancing the possibility of treatment or prognosis prediction for a neurodegenerative disease such as ALS.

DCTD is a gene that encodes a protein (dCMP deaminase) that contributes to deamination of dCMP to dUMP. An expression change of DCTD can contribute to regulation of nucleic acid biosynthesis. It has been found that an expression level of a gene product of this gene increases in the presence of TDP-43 mutant protein, which is a pathological condition of a neurodegenerative disease, and further, decreases in the presence of edaravone.

The upregulation of DCTD can lead to an imbalance of substrates or products in nucleic acid biosynthesis, potentially making DNA mutations more likely to occur during DNA replication. Therefore, it is considered that decreasing the expression level of DCTD is useful in contributing to the formation or functional maintenance of normal neuronal cells, suppressing the occurrence of cellular damage, or enhancing the possibility of treatment or prognosis prediction for a

neurodegenerative disease such as ALS.

MT2A is a gene that encodes a type of protein that belongs to a metallothionein family. An expression change of MT2A can contribute to heavy metal metabolism, such as intracellular heavy metal concentration, or apoptosis. It has been found that an expression level of a gene product of this gene increases in the presence of TDP-43 mutant protein, which is a pathological condition of a neurodegenerative disease, and further, decreases in the presence of edaravone.

The upregulation of MT2A is considered to be caused in part by an oxidative stress. Therefore, it is considered that decreasing the expression level of MT2A is useful in contributing to the normal functional maintenance of neuronal cells by adjusting response to an oxidative stress, suppressing the occurrence of cellular damage, or enhancing the possibility of treatment or prognosis prediction for a neurodegenerative disease such as ALS.

ASF1B is a gene that encodes a type of protein belonging to a histone chaperone H3/H4 family. An expression change of ASF1B can contribute to regulation of cell division, proliferation, and aging. It has been found that an expression level of a gene product of this gene increases in the presence of TDP-43 mutant protein, which is a pathological condition of a neurodegenerative disease, and further, decreases in the presence of edaravone.

An increase in expression level of ASF1B can contribute to occurrence or progression of a neurodegenerative disease such as ALS through some mechanism related to cell division or proliferation. Therefore, it is considered that decreasing the expression level of ASF1B is useful in suppressing the occurrence of cellular damage or enhancing the possibility of treatment or prognosis prediction for a neurodegenerative disease such as ALS.

FCSK is a gene that encodes a type of protein belonging to a phosphotransferase family and using alcohol as a receptor. An expression change of FCSK can contribute to metabolism of fructose and mannose. It has been found that an expression level of a gene product of this gene increases in the presence of TDP-43 mutant protein, which is a pathological condition of a neurodegenerative disease, and further, decreases in the presence of edaravone.

An increase in expression level of FCSK can contribute to occurrence or progression of a neurodegenerative disease such as ALS through some mechanism related to intracellular glucose metabolism. Therefore, it is considered that decreasing the expression level of FCSK is useful in suppressing the occurrence of cellular damage or enhancing the possibility of treatment or prognosis prediction for a neurodegenerative disease such as ALS.

MAST1 is a gene that encodes a type of serine/threonine protein kinase. An expression change of MAST1 is mainly related to brain development and can contribute to expression at postsynapses or synaptic terminals of neuromuscular junctions. It has been found that an expression level of a gene product of this gene increases in the presence of TDP-43 mutant protein, which is a pathological condition of a neurodegenerative disease, and further, decreases in the presence of edaravone.

It is considered that decreasing the expression level of MAST1 is useful in suppressing the occurrence of cellular damage by suppressing the formation of TDP-43 protein aggregates through phosphorylation, or enhancing the possibility of treatment or prognosis prediction for a neurodegenerative disease such as ALS.

FAIM2 is a gene that encodes a type of anti-apoptotic protein that protects cells from Fas-induced apoptosis. This gene is also known as protein lifeguard 2-like (LOC102551901) as a rat orthologue. An expression change of FAIM2 can contribute to a size of the cerebellum or a thickness of the internal granular layer, and development of Purkinje cells. It has been found that an expression level of a gene product of this gene decreases in the presence of TDP-43 mutant protein, which is a pathological condition of a neurodegenerative disease, and further, increases in the presence of edaravone.

Since apoptosis can be induced during neuronal damage, it is considered that the expression level of FAIM2 is decreased. Therefore, it is considered that increasing the expression level of FAIM2 is useful in enhancing the possibility of treatment or prognosis prediction for a neurodegenerative disease such as ALS by maintaining the survival of neuronal cells.

Further, FAIM2 is associated with the regulation of autophagy. Therefore, increasing the expression level of FAIM2 can suppress the formation of mutant SOD1 (Superoxide dismutase 1) protein aggregates, which is a pathological condition of a neurodegenerative disease such as ALS, or promote the degradation of aggregates of abnormal proteins such as TDP-43 and SOD1. As a result, it is considered that it is useful in enhancing the possibility of treatment or prognosis prediction for a neurodegenerative disease such as ALS.

An exposure method of the above-described composition is not particularly limited. Examples of the exposure method include administration to a living body, contact with a sample, and the like.

In the case of in vivo administration to a human or a non-human animal, the examples include oral administration and parenteral administration. In this case, the edaravone-containing composition is preferably a pharmaceutical composition containing edaravone.

Examples of parenteral administration include intravenous, intramuscular, intraperitoneal, subcutaneous or intradermal injection, injection into the digestive tract, inhalation, and the like. These administrations may be a single rapid administration or multiple rapid administrations, or may be a continuous administration such as infusion.

Contact with a sample can be performed, for example, using a liquid in which the above-described composition is dissolved or dispersed.

The above-described composition can be in a solid or liquid state at 1 atmosphere and 20 °C, depending on an

intended usage.

Examples of dosage forms when the composition is in a solid state include tablets, capsules, powders, fine granules, granules, suppositories, and the like.

Examples of dosage forms when the composition is in a liquid state include liquid agents, syrups, suspensions, injectables, infusions, and the like.

A liquid can be a solution containing a solvent or a dispersion containing a dispersing agent.

The above-described composition may further contain additives as needed. These additives are preferably pharmaceutically acceptable.

In one embodiment, when an additive is contained in the composition, the additive can constitute a remainder of the composition either as a single type of additive alone or as a combination of two or more types of additives.

As the additives, those used in the present technical field can be used without particular restrictions. As the additives, for example, excipients, disintegrants or disintegration aids, binders, lubricants, coating agents, colorants, diluents, vehicles, solvents or solubilizing agents, isotonic agents, pH adjusters, stabilizers, propellants, adhesives, and the like can be used. These additives can each be used independently, or two or more of the additives can be used in combination.

As vehicles or diluents, for example, water, electrolyte-containing water such as saline, monovalent alcohols with 1 to 3 carbon atoms such as methanol, ethanol, and propanol, or aqueous liquids used for cell culture can be used. These aqueous liquids can each be used independently or two or more of the aqueous liquids can be used in combination, as solvents or dispersing agents.

The content of edaravone in the composition can be appropriately changed depending on an applicable target. However, taking as an example the case of administration to a living mammal, regardless of the form of administration, the content of edaravone can be set to an amount that can be administered in a range of preferably 0.01 μg/kg body weight or more and 10 mg/kg body weight or less per day.

In one embodiment, taking as an example the case of administration to an adult human, regardless of the form of administration, the content of edaravone in the composition can be set to an amount that can be administered per day in a range of preferably 10 mg or more and 150 mg or less, more preferably 20 mg or more and 120 mg or less, and even more preferably 90 mg or more and 120 mg or less.

The above content can also be set as, for example, a therapeutically effective amount.

When exposing samples such as tissues or cells collected from animals or in vitro samples, the content of edaravone in the composition, taking a liquid form as an example, is preferably 0.1 μmol/L or more and 1000 μmol/L or less, more preferably 0.5 μmol/L or more and 500 μmol/L or less, even more preferably 0.75 μmol/L or more and 300 μmol/L or less, still more preferably 10 μmol/L or more and 300 μmol/L or less, and most preferably 50 μmol/L or more and 250 μmol/L or less.

In any form, when the content of edaravone is in the above-described ranges, occurrence of an adverse effect such as cellular damage, or progression of a disease to a target can be effectively suppressed.

The above is a description about the embodiments of the edaravone-containing composition. In the following, other embodiments of the present invention are described. In the following description, contents that differ from the embodiments described above will be mainly described, and for contents that are not specifically described, the matters described in the present specification will be applied as appropriate as long as the effects of the present invention are achieved.

In one embodiment, whether or not there is a possibility that a target has a disease can be evaluated by using an expression level of a gene product of a specific gene described above or a change in the expression level as an indicator. That is, the present embodiment relates to a method for evaluating a possibility of having a disease. By adopting this evaluation method, early detection of a disease or assessment of the need for appropriate treatment can be facilitated, potentially contributing to the improvement of a patient's quality of life.

In one embodiment, a disease that can be evaluated using the present method is preferably a neurodegenerative disease, more preferably ALS, and even more preferably ALS associated with the presence of TDP-43 mutant protein or abnormal intracellular localization of TDP-43.

In the present specification, when describing an embodiment of a method, unless otherwise specified, the method may include performing only one step, or may include performing multiple steps at the same time or in any order. Further, in a case of an embodiment that can include multiple steps, unless otherwise specified, one or more other steps may be further interposed between two steps, two steps may be performed consecutively such that no other steps are interposed therebetween, or two or more steps may be performed at the same time. Further, as long as the effects of the present invention are achieved, the steps according to the embodiments described in the present specification can be appropriately combined.

Specifically, the present method includes a step of evaluating whether or not there is a possibility that a target has a disease based on an expression level, or a changed in the expression level, of a gene product of one or more genes among specific genes in the target.

An applicable target of the present method is preferably a mammal or a sample derived from the mammal. More specifically, an applicable target of the present method is preferably a brain of a mammal or a sample derived from a brain.

Further, as an applicable target of the present method, a sample collected from a living or dead mammal can be preferably used, and the mammal is more preferably a human or a non-human animal.

In one embodiment, the present method can also be performed as a method to assist in detecting a disease from a target, and the disease is preferably a neurodegenerative disease.

In one embodiment, the present method can also be performed under an in vitro condition.

In one embodiment, the present method includes a step A1 of measuring an expression level of a gene product of a specific gene in a target. That is, in the present step A1, an expression level of a transcription product of, or a translation product encoded by, one or more specific genes selected from KAZALD1, SBK1, SCN2A, UBE2L6, ALPL, NTM, PTTG1, ITGB4, HAUS4, DCTD, MT2A, ASF1B, FCSK, MAST1 and FAIM2 is measured. In the present method, one or more types of gene products of specific genes can be used as indicators of a possibility of having diseases such as neurodegenerative diseases or as biomarkers for evaluation.

The measurement of an expression level may be performed in vitro or in vivo.

When quantifying a transcription product as a target as an expression level of a gene product, for example, RNA can be extracted from a sample as a measurement target using a conventional method, and, when necessary, after obtaining cDNA, which is a reverse transcription product, measurement can be performed using methods commonly known in the present technical field, such as Northern blot, RT-PCR, nucleic acid array, and RNA-seq. Further, when necessary, correction of a measured value can be performed based on expression levels of housekeeping genes such as GAPDH and β-actin.

When quantifying a translation product (that is, protein) as a target as an expression level of a gene product, measurement can be performed using immunological methods commonly known in the present technical field, such as ELISA, flow cytometry, Western blot method, or immunohistochemical staining, for example, using a sample or its solution as a measurement target. When necessary, correction of a measured value can be performed based on the protein expression levels of the housekeeping genes described above.

As other methods for quantifying a translation product, mass spectrometry, high-performance liquid chromatography, gas chromatography, NMR analysis, or a combination of these methods can be used. Examples of additional methods for measuring a translation product as a target include measuring the activity of the translation product and considering the measured activity as an expression level, or administering in vivo a ligand that specifically binds to the translation product.

In one embodiment, the present method includes, in addition to the step A1, a step B1 of evaluating whether or not there is a possibility that a target has a disease based on a measured expression level of a gene product or a change in the expression level.

In order to evaluate the possibility of a disease in the step B1, for example, a method including at least one of the following steps B11, B12, and B13 can be adopted.

In one embodiment, the present method can include, for example, performing at least one of the steps B12 and B13 after the step A1.

In one embodiment, the present method can include, for example, performing the step B11 before or after the step A1 or simultaneously with the step A1, and then performing the steps B12 and B13 in this order.

The step B11 is a step of using a normal target, that is, a healthy animal not affected by a disease or a sample derived from such an animal, as a control group, to measure an expression level of a gene product in the sample. The expression level of the gene product in the control group can also be used as a reference value.

The step B12 is a step of comparing the expression level of the gene product in the normal target (control group) with an expression level of a gene product in an evaluation target.

The step B13 is a step of comparing the normal target (control group) with the evaluation target, and evaluating that the evaluation target has a high possibility of having a disease when a change in expression level of a gene product of one or more genes among specific genes is confirmed.

The evaluation of the change in expression level is preferably performed based on the presence or absence of a statistically significant difference from a point of view of improving the evaluation accuracy.

The comparison of expression levels between experimental groups can be performed by comparing transcription products derived from the same gene or translation products derived from the same gene, under the same or similar experimental conditions.

Specifically, as shown in the examples to be described later, an expression level of a gene product of one or more genes belonging to the gene group (A) among the specific genes described above can increase compared to the normal target due to the presence of a disease (for example, a neurodegenerative disease). Therefore, when an expression level of a gene product of one or more genes belonging to the gene groups (A) is increased compared to the expression level in the normal target, it can be evaluated that the evaluation target or an animal from which the target is obtained has a high possibility of having a disease (for example, a neurodegenerative disease).

On the other hand, when an expression level of a gene product of one or more genes belonging to the gene group (A) among the specific genes described above is decreased or remains the same compared to the expression level in the normal target, it can be evaluated that the evaluation target or an animal from which the target is obtained has a low possibility of having a disease (for example, a neurodegenerative disease).

From a point of view of improving the evaluation accuracy regarding the possibility of having a disease and making it easier to exclude the possibility of a disease other than the one being evaluated, it is preferable to performed the evaluation by comparing expression levels of gene products with two or more genes among the specific genes as targets, and it is more preferable to perform the evaluation by comparing expression levels of gene products with all the specific genes described above as targets.

Further, an expression level of a gene product of genes belonging to the gene group (B) among the specific genes described above can decrease compared to the normal target due to the presence of a disease. Therefore, when an expression level of a gene product of the genes belonging to the gene groups (B) is decreased compared to the expression level in the normal target, it can be evaluated that the evaluation target or an animal from which the target is obtained has a high possibility of having a disease (for example, a neurodegenerative disease).

On the other hand, when an expression level of a gene product of the gene group (B) is increased or remains the same compared to the expression level in the normal target, it can be evaluated that the evaluation target or an animal from which the target is obtained has a low possibility of having a disease (for example, a neurodegenerative disease).

From a point of view of further improving the evaluation accuracy, it is preferable to perform the evaluation by comparing expression levels of gene products with all the genes belonging to the gene groups (A) and (B) as targets.

Alternatively or additionally, the possibility of the presence or absence of a disease can also be evaluated based on a cutoff value of an expression level of a gene product by setting the cutoff value as a reference value or a threshold. For example, when an expression level of a gene product of one or more genes among the specific genes described above is increased compared to the cutoff value (the gene group (A)) or decreased compared to the cutoff value (the gene group (B)), it can be evaluated that the evaluation target or an animal from which the target is derived has a high possibility of having a disease (for example, a neurodegenerative disease).

The "cutoff value" can be set, for example, as an expression level that satisfies, at a predetermined level, at least one of sensitivity and specificity when the presence or absence or severity of a disease is evaluated based on the cutoff value. Further, an expression level of a gene product in a healthy animal or a healthy human can also be used as the cutoff value. The cutoff value can be appropriately set according to the type of disease or the degree of strictness of the evaluation. Further, in order to evaluate the severity of a disease, multiple cut-off values may be set to stratify the possibility of having a disease.

In the following, still other embodiments are described. Also in the following description, contents that differ from the embodiments described above will be mainly described, and for contents that are not specifically described, the matters described in the present specification will be applied as appropriate as long as the effects of the present invention are achieved.

In one embodiment, whether or not there is a possibility that a target has responsiveness to edaravone can be evaluated by using an expression level of a gene product of a specific gene described above or a change in the expression level as an indicator. That is, the present embodiment relates to a method for evaluating responsiveness to edaravone. This method allows for earlier and simpler evaluation and prediction of the effects of edaravone on a disease.

An applicable target of the present method is preferably a target that has or is suspected of having a neurodegenerative disease. The applicable target is more preferably a patient with a neurodegenerative disease or a mammal that serves as a disease model, or a sample derived from such a mammal. In this case, the mammal is more preferably a human or a non-human animal. More specifically, the applicable target of the present evaluation method is preferably a sample collected from a patient, or a brain of a mammal or a sample derived from a brain.

Further, the disease as an applicable target of the present method is preferably a neurodegenerative disease, more preferably ALS, and even more preferably ALS accompanied by the presence of TDP-43 mutant protein or abnormal intracellular localization of TDP-43.

Specifically, the present method preferably uses a target that has or is suspected of having a disease and has been exposed to edaravone. The method includes a step of evaluating whether or not there is a possibility that a target has responsiveness to edaravone based on a change in expression level of a gene product of one or more genes among specific genes in the target.

In one embodiment, the present method can also be used as a method to assist in predicting whether or not a target (for example, a target that has or is suspected of having a neurodegenerative disease) will effectively respond to treatment with edaravone.

In one embodiment, the present method can also be performed under an in vitro condition.

In the following, in the present embodiment, an experimental group to which a target that has or is suspected of having a disease and has been exposed to edaravone belongs is also referred to as an "exposed disease group."

In one embodiment, the present method includes a step A2 of measuring an expression level of a gene product of

a specific gene in a target (exposed disease group) that has or is suspected of having a disease and has been exposed to edaravone. That is, in the present step, an expression level of a transcription product of, or a translation product encoded by, one or more specific genes selected from KAZALD1, SBK1, SCN2A, UBE2L6, ALPL, NTM, PTTG1, ITGB4, HAUS4, DCTD, MT2A, ASF1B, FCSK, MAST1, and FAIM2 in a target that has or is suspected of having a disease and has been exposed to edaravone is measured.

The measurement of the expression level of the transcription product or the translation product can be performed using the same measurement methods as in the embodiments described above.

Whether or not a target used in the present method has or is suspected of having a disease can be evaluated using, for example, the method in the embodiments described above. Additionally or alternatively, when performing evaluation regarding a neurodegenerative disease as one form of disease, it is also possible to evaluate that a target has or is suspected of having a neurodegenerative disease based on the presence of TDP-43 mutant protein or based on abnormal intracellular localization of TDP-43. The presence of TDP-43 mutant protein or the abnormal intracellular localization of TDP-43 can be determined, for example, by subjecting a sample such as tissue or cells to an immunological method described above.

Further, for example, animals including humans that have been evaluated as having or suspected of having neurodegenerative diseases by performing examinations such as electromyography on their living bodies, or samples from such animals, can be used.

Exposure of a target to edaravone may be performed, for example, by in vivo administration, or after collecting a sample from an animal that has not been exposed to edaravone, the sample may be brought into contact with tedaravone under an in vitro condition.

In the case where edaravone has been administered in vivo, for example, a sample collected from a living body such as a human patient or an animal after the administration may be subjected to expression level measurement. In this case, the sample is treated as target that has been exposed to edaravone.

In either case, the exposure to edaravone may use edaravone itself, or may use a composition according to the embodiments described above.

An exposure amount of a target to edaravone can be appropriately changed depending on a purpose. For example, in the case of in vivo administration, the exposure amount of edaravone can be set to a range of preferably 0.01 μg/kg body weight or more and 10 mg/kg body weight or less per day.

In one embodiment, taking as an example the case of administration to an adult human, regardless of the form of administration, the exposure amount of edaravone can be set to an amount that can be administered per day in a range of preferably 10 mg or more and 150 mg or less, more preferably 20 mg or more and 120 mg or less, and even more preferably 90 mg or more and 120 mg or less.

For the in vivo administration, for example, the oral and/or parenteral administration methods described above can be adopted.

When exposing samples such as tissues or cells collected from animals or in vitro samples, an exposure concentration of edaravone is preferably 0.1 μmol/L or more and 1000 μmol/L or less, more preferably 0.5 μmol/L or more and 500 μmol/L or less, even more preferably 0.75 μmol/L or more and 300 μmol/L or less, still more preferably 10 μmol/L or more and 300 μmol/L or less, and most preferably 50 μmol/L or more and 250 μmol/L or less.

An exposure time of a sample to edaravone can be appropriately changed depending on a purpose. For example, the exposure time can be preferably 1 hour or more and 120 hours or less, and more preferably 12 hours or more and 60 hours or less, provided that the concentration is as described above.

In the present method, from a point of view of further improving the evaluation accuracy, the target may be further exposed to a stress inducer simultaneously with, or before or after, the exposure to edaravone.

Examples of stress inducers include compounds that cause a stress in an exposure target, making it more susceptible to cellular damage. Examples of such compounds include one or more of endoplasmic reticulum stress agents, osmotic stress agents, oxidative stress agents, and the like. These are preferably used in an in vitro environment.

Examples of endoplasmic reticulum stress agents include thapsigargin, tunicamycin, dithiothreitol, and the like.

Examples of osmotic stress agents include sorbitol, sodium chloride, and the like.

Examples of oxidative stress agents include ethacrynic acid, arsenite such as sodium arsenite, quinone compounds such as tert-butylhydroquinone, and the like.

When stress inducers are included, the contents of stress inducers can be appropriately changed depending on the types and combination of the compounds, but can each be independently set to, for example, 0.1 μmol/L or more and 1000 μmol/L or less, for example, 1 μmol/L or more and 100 μmol/L or less.

An exposure time of a stress inducer can be in the same range as the exposure time of edaravone.

In one embodiment, the present method includes, in addition to the step A2, a step B2 of evaluating whether or not there is a possibility that a target has responsiveness to edaravone based on a measured change in expression level of a gene product.

In order to evaluate the possibility of responsiveness to edaravone in the step B2, for example, a method

including at least one of the following steps B21, B22, and B23 can be adopted.

In one embodiment, the present method can include, for example, performing at least one of the steps B22 and B23 after the step A2.

In one embodiment, the present method can include, for example, performing the step B21 before or after the step A2, or simultaneously with the step A2, and then performing the steps B22 and B23 in this order.

The step B21 is a step of measuring an expression level of a gene product in an unexposed disease group, using a target that has or is suspected of having a disease and has not been exposed to edaravone as the unexposed disease group. From a point of view of improving the evaluation accuracy, it is preferable that the target in the unexposed disease group and the target in the exposed disease group are the same except for the presence or absence of exposure to edaravone.

The step B22 is a step of comparing the expression level of the gene product in the target belonging to the unexposed disease group with the expression level of the gene product in the evaluation target (the exposed disease group).

The step B23 is a step of comparing the unexposed disease group with the exposed disease group, and evaluating that the target has a high possibility of having responsiveness to edaravone when a change in expression level of a gene product of one or more genes among specific genes is confirmed.

The evaluation of the change in expression level may be performed by setting an arbitrary threshold, or may be performed based on a statistically significant difference. The evaluation of the change in expression level is preferably performed based on the presence or absence of a statistically significant difference from a point of view of improving the evaluation accuracy.

Specifically, an expression level of a gene product of one or more genes belonging to the gene group (A) among the specific genes described above can increase in the presence of a disease (for example, a neurodegenerative disease) compared to a normal target, and can further decrease by exposure to edaravone compared to the case where only a disease (for example, a neurodegenerative disease) is present. Therefore, for one or more genes in the gene group (A), when an expression level of a gene product in the exposed disease group is decreased compared to an expression level of a gene product in the unexposed disease group, it can be evaluated that a target that has or is suspected of having a disease (for example, a neurodegenerative disease) has a high possibility of having responsiveness to edaravone.

On the other hand, for the gene group (A), when an expression level of a gene product in the exposed disease group is increased or remains the same compared to an expression level of a gene product in the unexposed disease group, it can be evaluated that a target that has or is suspected of having a disease (for example, a neurodegenerative disease) has a low possibility of having responsiveness to edaravone.

From a point of view of improving the evaluation accuracy regarding the responsiveness to edaravone, it is preferable to performed the evaluation by comparing expression levels of gene products with multiple genes among the specific genes as targets, and it is more preferable to perform the evaluation by comparing expression levels of gene products with all the specific genes described above as targets.

Further, an expression level of a gene product of genes belonging to the gene group (B) among the specific genes described above can decrease in the presence of a disease (for example, a neurodegenerative disease) compared to a normal target, and can further increase by exposure to edaravone compared to the case where only a disease (for example, a neurodegenerative disease) is present. Therefore, for genes belonging to the gene group (B), when an expression level of a gene product in the exposed disease group is increased compared to an expression level of a gene product in the unexposed disease group, it can be evaluated that a target that has or is suspected of having a disease (for example, a neurodegenerative disease) has a high possibility of having responsiveness to edaravone.

On the other hand, for the gene group (B), when an expression level of a gene product in the exposed disease group is decreased or remains the same compared to an expression level of a gene product in the unexposed disease group, it can be evaluated that a target that has or is suspected of having a disease (for example, a neurodegenerative disease) has a low possibility of having responsiveness to edaravone.

From a point of view of further improving the evaluation accuracy, it is preferable to perform the evaluation by comparing expression levels of gene products with all the genes belonging to the gene groups (A) and (B) as targets.

From a point of view of more accurately evaluating the possibility of responsiveness to edaravone, it is also preferable to further use a normal target, that is, a healthy animal not affected by a disease or a sample derived from such an animal, as a control group and to perform comparison with an expression level of a gene product in the evaluation target. That is, in the present embodiment, it is preferable to perform the comparison using two or three experimental groups among the exposed disease group, the unexposed disease group, and the normal group.

An expression level of a gene product of the specific gene described above can be increased or decreased in the presence of a disease compared to a normal target, and further can be decreased or increased by exposure to edaravone. Based on such a useful expression profile, the present embodiment can evaluate the possibility of a therapeutic effect of edaravone administration or assist in effective prediction of therapeutic responsiveness.

Specifically, the step B2 in the present embodiment preferably includes at least one of the following steps B24,

B25, and B26.

In one embodiment, the present method can include, for example, performing at least one of the steps B25 and B26 after the step A2.

In one embodiment, the present method can include, for example, performing the step B24 before or after the step A2, or simultaneously with the step A2, and then performing the steps B25 and B26 in this order.

The step B24 is a step for measuring an expression level of a gene product in a normal target (control group). The normal target is preferably not exposed to edaravone.

The step B25 is a step of comparing an expression level of a gene product in a target that has or is suspected of having a disease and has been exposed to edaravone (exposed disease group) with an expression level of a gene product in a normal target (control group).

The step B26 is a step of evaluating that there is a possibility that a target that has or is suspected of having a disease has responsiveness to edaravone and edaravone is highly effective on the target, when no change in an expression level of a gene product in a target in an exposed disease group is confirmed compared to an expression level of a gene product in a normal target for one or more specific genes. When evaluated in this way, for example, it can also be evaluated that there is a possibility that an exposure amount or exposure frequency of edaravone in an evaluation target is appropriate.

On the other hand, when a change in an expression level of a gene product in a target in an exposed disease group is confirmed compared to an expression level of a gene product in a normal target for one or more specific genes, it can be evaluated that there is a possibility that an evaluation target does not have responsiveness to edaravone, or it can be evaluated that there is a possibility that the responsiveness to edaravone is low and the effect of edaravone is low, or the responsiveness to edaravone is high and the effect of edaravone is very high. When evaluated in this way, depending on an increase or decrease in expression level or a degree of the increase or decrease, it can also be evaluated that, for example, there is a possibility that an exposure amount or exposure frequency of edaravone in an evaluation target is excessive or insufficient.

Further, taking the gene group (A) as an example, when an expression level of a gene product in the exposed disease group is lower than an expression level of a gene product in the unexposed disease group and higher than an expression level of a gene product in a normal target (control group), it can also be evaluated that there is a possibility that an evaluation target has low responsiveness to edaravone or there is a possibility that an exposure amount or exposure frequency of edaravone is insufficient.

Based on such evaluation, for example, a step of determining whether or not there is a need for appropriate treatment of a target may be further performed to allow monitoring of the effectiveness of edaravone treatment or assisting in treatment optimization. Examples of such a need include increasing or decreasing the exposure amount or exposure frequency of edaravone, or implementing measures other than exposure to edaravone.

In any case, the evaluation of the change in expression level may be performed by setting an arbitrary threshold, or may be performed based on a statistically significant difference. The evaluation of the change in expression level is preferably performed based on the presence or absence of a statistically significant difference from a point of view of improving the evaluation accuracy.

As another embodiment of a method for evaluating edaravone responsiveness, for example, the step B2 in the present embodiment preferably includes the steps B21 and B24, as well as the following steps B27 and B28.

In one embodiment, the present method can include, for example, performing the steps A1, B21 and B24 simultaneously or in any order, and then performing the steps B27 and B28 in this order.

The step B27 is a step of comparing an expression level of a gene product of a specific gene in a healthy individual (control group) with an expression level of a gene product of a specific gene in an unexposed disease group.

The step B28 is a step of evaluating that a target in the unexposed disease group or a patient or animal from which the target is sampled has a high possibility of responding to treatment with edaravone, when it is determined that there is a change in expression level between the two groups.

That is, the present embodiment is an example of a method for distinguishing the responsiveness to edaravone using, as a biomarker, an expression level of a gene product of a specific gene that has increased or decreased due to a disease compared to a healthy individual.

In one embodiment, the present method can also be used as a method for assisting in prediction of whether or not a target that has or is suspected of having a disease will effectively respond to treatment with edaravone.

When comparing the control group with the unexposed disease group, when an expression level of a gene product in the unexposed disease group is higher than an expression level of a gene product in the normal group for one or more genes in the gene group (A), it can be evaluated that a target that has or is suspected of having a disease (for example, a neurodegenerative disease) has a high possibility of having responsiveness to edaravone.

On the other hand, when comparing the control group with the unexposed disease group, when an expression level of a gene product in the unexposed disease group is lower than or equal to an expression level of a gene product in the normal group for the gene group (A), it can be evaluated that a target that has or is suspected of having a disease (for

example, a neurodegenerative disease) has a low possibility of having responsiveness to edaravone.

Similarly, for genes belonging to the gene group (B), when comparing the control group with the unexposed disease group, when an expression level of a gene product in the unexposed disease group is lower than an expression level of a gene product in the normal group, it can be evaluated that a target that has or is suspected of having a disease (for example, a neurodegenerative disease) has a high possibility of having responsiveness to edaravone.

On the other hand, when comparing the control group with the unexposed disease group, when an expression level of a gene product in the unexposed disease group is higher than or equal to an expression level of a gene product in the normal group for the gene group (B), it can be evaluated that a target that has or is suspected of having a disease (for example, a neurodegenerative disease) has a low possibility of having responsiveness to edaravone.

In the following, still other embodiments are described. Also in the following description, contents that differ from the embodiments described above will be mainly described, and for contents that are not specifically described, the matters described in the present specification will be applied as appropriate as long as the effects of the present invention are achieved.

In one embodiment, by using an expression level of a gene product of a specific gene described above or a change in the expression level as an indicator, any candidate substance can be selected as a substance capable of treating or preventing a neurodegenerative disease. That is, the present embodiment relates to a method for screening a substance capable of treating or preventing a neurodegenerative disease. With this method, it is possible to determine, using a method different from previous methods, whether an unknown substance is a substance capable of treating or preventing a neurodegenerative disease. As a result, it is possible to screen the substance as a drug candidate at an earlier stage, contributing to the early development of pharmaceuticals.

Specifically, the method for screening a substance capable of treating or preventing a neurodegenerative disease includes: a step A3 of exposing a target to a test substance; and a step B3 of selecting a test substance as a substance capable of treating or preventing a neurodegenerative disease based on a change in expression level of a gene product of a specific gene in a target exposed to the test substance.

In the present method, it is preferable that edaravone is excluded from the test substances. That is, it is preferable that a target used for substance evaluation in the present method is exposed to a test substance but is not exposed to edaravone.

In the present method, a target to be exposed to a test substance is preferably a mammal that has or is suspected of having a neurodegenerative disease, or a sample derived from a mammal that has or is suspected of having a neurodegenerative disease, and more preferably the mammal is a human. More specifically, an applicable target of the present method is preferably a brain of a mammal or a sample derived from a brain.

Further, the neurodegenerative disease as an evaluation target is preferably ALS, and more preferably ALS accompanied by the presence of TDP-43 mutant protein or abnormal intracellular localization of TDP-43.

The test substance is not particularly limited and examples thereof include organic low molecular weight compounds, nucleic acids, proteins, peptides, antibodies, natural compounds, and the like.

In the step A3, the method for exposing a target to a test substance is not particularly limited and can be performed in vitro or in vivo, for example, using a method similar to that in the embodiments described above.

When exposure of a target to a test substance is performed in vitro, for example, it can be performed by bring the target into contact with a liquid in which the test substance is dissolved or dispersed. Examples of the liquid include saline, buffer, or culture medium containing the test substance.

When exposure of a target to a test substance is performed in vivo, for example, the test substance itself or a liquid in which the test substance is dissolved or dispersed can be administered to a mammal. The administration method may be oral or parenteral.

In the step B3, an expression level of a gene product of a specific gene is measured, and based on a measured change in expression level, a test substance is determined to be a substance capable of treating or preventing a neurodegenerative disease.

In order to determine in the step B3 that a test substance is an intended substance, a method including at least one of the following steps B31, B32, and B33 can be adopted.

In one embodiment, the present method can include, for example, performing at least one of the steps B32 and B33 after the step A3.

In one embodiment, the present method can include, for example, performing the step B31 before or after the step A3, or simultaneously with the step A3, and then performing the steps B32 and B33 in this order.

The step B31 is a step of measuring an expression level of a gene product of a specific gene in an unexposed group, using a target that has not been exposed to a test substance and edaravone and has or is suspected of having a neurodegenerative disease as the unexposed group.

The step B32 is a step of comparing an expression level of a gene product in the unexposed group with an expression level of a gene product in a target that has been in contact with a test substance (test substance-exposed group).

The step B33 is a step of comparing the unexposed group and the exposed group, and when a change in expression level of the gene product is confirmed for one or more specific genes, determining that the test substance is a substance capable of treating or preventing a neurodegenerative disease and selecting the substance.

The determination of the change in expression level may be performed by setting an arbitrary threshold, or may be performed based on a statistically significant difference. The determination of the change in expression level is preferably performed based on the presence or absence of a statistically significant difference from a point of view of improving the determination accuracy.

An expression level of a gene product of one or more genes belonging to the gene group (A) among the specific genes described above can increase due to the presence of a neurodegenerative disease. Then, a test substance with similar efficacy to edaravone can decrease an expression level of a gene product in a target. Therefore, when an expression level of a gene product of one or more genes among the specific genes in the test substance-exposed group is decreased compared to an expression level in the unexposed group, it can be determined that the test substance is a substance capable of treating or preventing a neurodegenerative disease.

On the other hand, when an expression level of a gene product of one or more genes belonging to the gene group (A) among the specific genes in the test substance-exposed group is increased or remains the same compared to an expression level in the unexposed group, it can be determined that the test substance is not a substance capable of treating or preventing a neurodegenerative disease or such a possibility is low.

An expression level of a gene product of genes belonging to the gene group (B) among the specific genes described above can decrease due to the presence of a neurodegenerative disease. Then, a test substance with similar efficacy to edaravone can increase an expression level of a gene product in a target. Therefore, when an expression level of a gene product of the genes in the test substance-exposed group is increased compared to an expression level in the unexposed group, it can be determined that the test substance is a substance capable of treating or preventing a neurodegenerative disease.

On the other hand, when an expression level of a gene product of the genes belonging to the gene group (B) in the test substance-exposed group is decreased or remains the same compared to an expression level in the unexposed group, it can be determined that the test substance is not a substance capable of treating or preventing a neurodegenerative disease or such a possibility is low.

From a point of view of improving the accuracy and reproducibility of the determination of the test substance and performing the screening more effectively, it is preferable to perform the determination by comparing expression levels of gene products of multiple genes among the specific genes, and it is more preferable to perform the determination by comparing all the specific genes described above.

From a point of view of more accurately determining a degree of contribution of the test substance to the treatment or prevention of a disease, the step B3 preferably includes a step B35 of further using a target that has not been exposed to the test substance but has been exposed to edaravone as an edaravone-exposed group, and comparing with an expression level of a gene product in the test substance-exposed group. That is, it is preferable to compare expression levels in each target using three experimental groups including the test substance-exposed group, the edaravone-exposed group, and the unexposed group. That is, in the present embodiment, the unexposed group can be used as a negative control, and the edaravone-exposed group can be used as a positive control.

The targets used in these three experimental groups are preferably mammals that have or are suspected of having a neurodegenerative disease, or samples derived from mammals that have or are suspected of having a neurodegenerative disease. More specifically, applicable targets of the present determination method are preferably brains of mammals or samples derived from brains of mammals.

In this case, preparing groups with the same concentrations of the test substance and edaravone to be exposed is preferable in improving the accuracy and reproducibility of the evaluation.

In one embodiment of the present method, as the step B34, a step of measuring an expression level of a gene product of a specific gene in the edaravone-exposed group is performed. When the step B34 is performed, it may be performed before the step B35.

In one embodiment, the present method can include performing the steps A3, B31, and B34 simultaneously or in any order, and then performing (a) the steps B32 and B33 in this order, and/or (b) the step B35. When performing both the step groups (a) and (b), at least the step B32 may be performed before the steps B33 and B35, and the steps B33 and B35 can be performed in any order or at the same time.

The step B35 is a step of comparing an expression level of a gene product in the test substance-exposed group with an expression level of a gene product in a target that has not been exposed to the test substance but has been exposed to edaravone.

Expression levels of gene products of the specific genes can increase or decrease due to the presence of a neurodegenerative disease. Then, a test substance with similar efficacy to edaravone can increase or decrease the expression levels of the gene products of the specific genes. Therefore, when no change in expression level of a gene product in a target that has been exposed to a test substance is confirmed compared to an expression level of a gene

product in a target that has been exposed to edaravone for one or more specific genes, the test substance can be selected as a substance capable of treating or preventing a neurodegenerative disease. Further, it can also be determined that the test substance has a high possibility of being a substance having efficacy equivalent to edaravone in treating or preventing a neurodegenerative disease.

On the other hand, when a change in expression level of a gene product in the test substance-exposed group is confirmed compared to an expression level of a gene product in the edaravone-exposed group for one or more specific genes, it can be determined that the test substance has a low possibility of being a substance capable of treating or preventing a neurodegenerative disease. Further, it can be determined that the test substance does not have efficacy equivalent to edaravone, or has a low possibility of having efficacy, in treating or preventing a neurodegenerative disease.

Further, for example, for one or more genes belonging to the gene group (A) among the specific genes, when an expression level of a gene product in the test substance-exposed group is decreased compared to an expression level of a gene product in the unexposed group and is increased compared to an expression level of a gene product in the edaravone-exposed group, it can be evaluated that there is a possibility that the efficacy of the test substance is less than the efficacy of edaravone. For the gene belonging to the gene group (B) among the specific genes, when an expression level of a gene product in the test substance-exposed group is increased compared to an expression level of a gene product in the unexposed group and is decreased compared to an expression level of a gene product in the edaravone-exposed group, it can be evaluated that there is a possibility that the efficacy of the test substance is less than the efficacy of edaravone. Based on such evaluations, measures can be taken to enhance the efficacy, such as changing the test substance used or adjusting the exposure amount or exposure frequency of the test substance.

In any case, the determination of the change in expression level is preferably performed based on the presence or absence of a statistically significant difference from a point of view of improving the determination accuracy.

In one embodiment, in order to determine in the step B3 that a test substance is an intended substance, for example, the determination can also be performed by comparing a result of an expression level in the test substance-exposed group and a result of an expression level in a normal target.

Specifically, in one embodiment, the step B3 preferably includes at least one of the following steps B36, B37, and B38.

In one embodiment, the present method can include, for example, performing at least one of the steps B37 and B38 after the step A3.

In one embodiment, the present method can include, for example, performing the step B36 before or after the step A3, or simultaneously with the step A3, and then performing the steps B37 and B38 in this order.

In one embodiment, the present method may adopt a combination of two or all of the steps B31, B32 and B33 described above, or a combination of the steps B34 and B35 described above.

The step B36 is a step of measuring an expression level of a gene product of a specific gene in a control group, using a normal target that has not been exposed to a test substance and edaravone as the control group.

The step B37 is a step of comparing an expression level of a gene product in a normal target (control group) with an expression level of a gene product in a target that has been in contact with a test substance and has or is suspected of having a neurodegenerative disease (test substance-exposed group).

The step B38 is a step of comparing a normal target (control group) and an exposed group, and when no change in expression level of a gene product is confirmed for one or more specific genes, determining that a test substance is a substance capable of treating or preventing a neurodegenerative disease and selecting the substance.

The determination of the change in expression level may be performed by setting an arbitrary threshold, or may be performed based on a statistically significant difference. The determination of the change in expression level is preferably performed based on the presence or absence of a statistically significant difference from a point of view of improving the determination accuracy.

An expression level of a gene product of one or more genes among the specific genes described above can increase or decrease due to the presence of a neurodegenerative disease. Therefore, when there is a possibility that a test substance is a substance capable of treating or preventing a neurodegenerative disease, an expression level of a gene product of one or more genes among these specific genes in a test substance-exposed group can decrease or increase.

Therefore, when no change in a gene product in a test substance-exposed group is confirmed compared to an expression level in a normal target for one or more specific genes, it can be determined that the test substance is a substance capable of treating or preventing a neurodegenerative disease.

On the other hand, when a change in a gene product in a test substance-exposed group is confirmed compared to an expression level in a normal target for one or more specific genes, it can be determined that the test substance is not a substance capable of treating or preventing a neurodegenerative disease or such a possibility is low.

Gene products of the specific genes described above can also be used as biomarkers. That is, the present embodiment relates to biomarkers.

As shown in the examples to be described below, the specific genes described above were newly identified by the present inventor as those showing a change in expression level based on results from exposure to edaravone and disease

models. Therefore, by using a gene product of a specific gene as a biomarker, it is possible to easily diagnose the presence or absence of a neurodegenerative disease, the progression or severity of the disease, or the possibility of the disease. Further, by using a gene product as a biomarker, it is possible to easily evaluate the possibility of contributing to the responsiveness to or efficacy of edaravone against a disease, or the efficacy of a test substance.

Specifically, in one embodiment, a biomarker includes a gene product of one or more of specific genes selected from KAZALD1, SBK1, SCN2A, UBE2L6, ALPL, NTM, PTTG1, ITGB4, HAUS4, DCTD, MT2A, ASF1B, FCSK, MAST1, and FAIM2. By including multiple types of gene products derived from two or more genes as a biomarker, the reliability of evaluation or diagnosis can be improved.

In one embodiment, the biomarker described above may consist solely of a gene product derived from a specific gene described above.

A gene product in the biomarker described above may consist solely of a transcription product, may consist solely of a translation product, or may consist of a combination of a transcription product and a translation product.

The biomarker described above can be used to determine the presence or possibility of a neurodegenerative disease in a target, or to predict prognosis.

In another embodiment, the biomarker described above is used for diagnosing prediction of responsiveness to edaravone.

In yet another embodiment, the biomarker described above is used for diagnosing responsiveness to a test substance.

For the biomarker, one or more types of the samples described above, more specifically, tissues, cells, and body fluids collected from living organisms such as animals regardless of whether or not edaravone has been administered, and animals regardless of whether or not they have a disease, can be used as a specimen, and at least one of a transcription product and a translation product in the specimen can be measured using the measurement method described above. Preferably, this can be performed in vitro.

The present disclosure also provides a detection kit for detecting the biomarker described above. The detection kit includes a detection reagent capable of specifically detecting a gene product of a specific gene. This kit can detect, preferably in vitro, one or more of the gene products as a biomarker.

Examples of the detection reagent include a nucleic acid probe or primer for detecting a transcription product of a specific gene. Other examples of the detection reagent include an antibody or antibody fragment or the like for detecting a translation product of a specific gene. The detection reagent may be labeled, for example, with a fluorescent substance or a radioactive nuclide, when necessary.

Further, the present disclosure also provides a method for treating a neurodegenerative disease using the composition described above. The treatment method may include, for example, orally or parenterally administering the composition described above to a human or a non-human animal. When an administration target is a human, the human is preferably a patient with a neurodegenerative disease.

In one embodiment, the treatment method described above can adopt administration conditions using, for example, edaravone injection or oral administration formulations as an edaravone-containing composition. For administration conditions of edaravone injection, for example, the administration method described in WO2020/091036, or the administration method using edaravone preparations used in clinical settings can be adopted.

Further, the present disclosure also provides the use of a substance that causes a change in expression level of a gene product of the specific genes described above in the manufacture of a composition for the prevention or treatment of a neurodegenerative disease.

Further, the present disclosure also provides a substance for use in causing a change in expression level of a gene product of the specific genes described above. **In** one embodiment, a substance is also provided for use in the treatment of a neurodegenerative disease by causing a change in expression level of a gene product of the specific genes described above.

The substance described above is not particularly limited, and examples thereof include edaravone, edaravone-containing compositions, organic low molecular weight compounds, nucleic acids, proteins, peptides, antibodies, natural components, and test substances found using the screening method described above.

The present disclosure also provides a method for suppressing the occurrence or progression of cellular damage by causing a change in expression level of a gene product of the specific genes described above in cells.

For causing a change in expression level, for example, a method for bringing cells into contact with a predetermined substance can be used. The substance used for the contact is not particularly limited, and examples thereof include edaravone, edaravone-containing compositions, organic low molecular weight compounds, nucleic acids, proteins, peptides, antibodies, natural components, and test substances found using the screening method described above.

The above-described embodiments disclosed in the present specification may each independently adopt one of the specific genes described above, or all of the specific genes, or any combination of two or more of the specific genes. The following combinations of the specific genes are examples, and it is not limited to these combinations.

For example, in one embodiment, the specific genes described above may be one or more selected from KAZALD1, SBK1, UBE2L6, ALPL, NTM, PTTG1, ITGB4, HAUS4, DCTD, MT2A, ASF1B, FCSK, MAST1 and FAIM2.

Further, for example, in one embodiment, the specific genes described above may be one or more selected from KAZALD1, SBK1, UBE2L6, NTM, HAUS4, DCTD, ASF1B, FCSK, and FAIM2.

Further, for example, in one embodiment, the specific genes described above may be one or more selected from KAZALD1, SBK1, UBE2L6, NTM, DCTD, ASF1B and FCSK.

Further, for example, in one embodiment, the specific genes described above may be one or more selected from KAZALD1, SBK1, DCTD and FCSK.

Further, for example, in one embodiment, the specific genes described above may be one or more selected from SBK1, DCTD and FCSK.

Further, for example, in one embodiment, the specific genes described above may be one or two selected from DCTD and FCSK.

Regarding the embodiments described above, the present specification further discloses the following embodiments.

<1> A composition for causing a change in expression level of a gene product in a target, the composition comprising edaravone, wherein the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2.

<2> The composition according to <1>, used as a pharmaceutical containing edaravone as an active ingredient.

<3> The composition according to<2>, used for treating or preventing a neurodegenerative disease.

<4> The composition according to <3>, wherein the neurodegenerative disease is amyotrophic lateral sclerosis (ALS).

<5> The composition according to any one of <1> - <4>, wherein the target is preferably a mammal or a sample derived from a mammal, and the mammal is more preferably a human or a rat.

<6> The composition according to any one of <1> - <5>, wherein the target is a brain of a mammal or a sample derived from a brain.

<7> The composition according to any one of <1> - <6>, used to decrease an expression level of a gene product of a first gene in the target having a neurodegenerative disease, and/or used to increase an expression level of a gene product of a second gene in the target having a neurodegenerative disease, wherein the first gene is one or more selected from KAZALD1, SBK1, SCN2A, UBE2L6, ALPL, NTM, PTTG1, ITGB4, HAUS4, DCTD, MT2A, ASF1B, FCSK and MAST1, and the second gene is FAIM2.

<8> The composition according to any one of <1> - <7>, wherein the target includes one or more selected from neuronal cells (neurons), glial cells, astrocytes and neural stem cells.

<9> The composition according to any one of <1> - <8>, wherein TDP-43 mutant protein is present in the target.

<10> A method for evaluating a possibility of having a neurodegenerative disease of a target, the method comprising a step $\alpha$ of evaluating whether or not there is a possibility that the target has a neurodegenerative disease based on a change in expression level of a gene product in the target, wherein the gene product is a gene product of one or more genes selected from KAZALD1, SBK1, UBE2L6, ALPL, NTM, PTTG1, ITGB4, HAUS4, DCTD, MT2A, ASF1B, FCSK, MAST1 and FAIM2.

<11> The method according to <10>, wherein the step $\alpha$ includes: a step $\alpha 1$ of comparing an expression level of the gene product in the target with an expression level of the gene product in a normal target; and a step $\alpha 2$ of evaluating that the target has a high possibility of having a neurodegenerative disease when there is a change in the expression level of the gene product between the target and the normal target.

<12> The method according to <11>, wherein the step $\alpha 2$ is a step of evaluating that the target has a high possibility of having a neurodegenerative disease when the expression level of the gene product of one or more genes selected from KAZALD1, SBK1, UBE2L6, ALPL, NTM, PTTG1, ITGB4, HAUS4, DCTD, MT2A, ASF1B, FCSK, and MAST1 is higher, or when the expression level of the gene product of FAIM2 is lower, compared to the expression level of the

gene product in the normal target.

<13> The method according to any one of <10> - <12>, wherein the gene is any one of the following (i) to (v):
(i) one or more selected from KAZALD1, SBK1, HAUS4, DCTD, FCSK and FAIM2, (ii) one or more selected from KAZALD1, SBK1, DCTD and FCSK, (iii) one or more selected from SBK1, DCTD and FCSK, (iv) one or more selected from DCTD and FCSK, and (v) one or more selected from KAZALD1, SBK1, UBE2L6, NTM, DCTD, ASF1B and FCSK.

<14> The method according to any one of <10> - <13>, wherein the target is a brain of a mammal or a sample derived from a brain, and the mammal is preferably a human.

<15> The method according to any one of <10> - <14>, wherein the target includes one or more selected from neuronal cells (neurons), glial cells, astrocytes and neural stem cells.

<16> The method according to any one of <10> - <15>, wherein the neurodegenerative disease is amyotrophic lateral sclerosis (ALS).

<17> A method for evaluating responsiveness of a target to edaravone, the method comprising a step β of evaluating whether or not there is a possibility that the target has responsiveness to edaravone based on a change in expression level of a gene product due to exposure of the target to edaravone, wherein the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2, and the target is preferably a target that has or is suspected of having a neurodegenerative disease.

<18> The method according to <17>, wherein the step β includes: a step β1 of comparing an expression level of the gene product in a first target that has or is suspected of having a neurodegenerative disease and has not been exposed to edaravone with an expression level of the gene product in a second target that has or is suspected of having a neurodegenerative disease and has been exposed to edaravone; and a step β2 of evaluating that a target has or is suspected of having a neurodegenerative disease has a high possibility of having responsiveness to edaravone when the expression level of the gene product has changed between the first target and the second target.

<19> The method according to <18>, wherein the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK and MAST1, and the step β2 is a step of evaluating that a target that has or is suspected of having a neurodegenerative disease has a high possibility of having responsiveness to edaravone when the expression level of the gene product in the second target is lower than the expression level of the gene product in the first target.

<20> The method according to any one of <18> and <19>, wherein the gene product is a gene product of FAIM2, and the step β2 is a step of evaluating that a target that has or is suspected of having a neurodegenerative disease ihas a high possibility of having responsiveness to edaravone when the expression level of the gene product in the second target is higher than the expression level of the gene product in the first target.

<21> The method according to any one of <17> - <20>, wherein the step β includes: a step β3 of comparing an expression level of the gene product in a second target that has or is suspected of having a neurodegenerative disease and has been exposed to edaravone with an expression level of the gene product in a normal target; and a step β4 of evaluating that there is a possibility that an effect due to edaravone is high for a target that has or is suspected of having a neurodegenerative disease when there is no change in the expression level of the gene product between the second target and the normal target.

<22> The method according to any one of <17> - <21>, wherein the step β includes: a step β3 of comparing an expression level of the gene product in a second target that has or is suspected of having a neurodegenerative disease and has been exposed to edaravone with an expression level of the gene product in a normal target; and a step β5 of evaluating that there is a possibility that an effect due to edaravone is low for a target that has or is suspected of having a neurodegenerative disease when there is a change in the expression level of the gene product between the second target and the normal target.

<23> The method according to any one of <17> - <22>, wherein the neurodegenerative disease is amyotrophic lateral sclerosis (ALS), and the target is a human or a sample derived from a human.

<24> A method for screening a substance capable of treating or preventing a neurodegenerative disease, the method comprising a step $\gamma$ of selecting a test substance as a substance capable of treating or preventing a neurodegenerative disease based on a change in expression level of a gene product in a target exposed to the test substance, wherein the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2.

<25> The method according to <24>, wherein the step $\gamma$ includes: a step $\gamma$1 of comparing an expression level of the gene product in a target that has or is suspected of having a neurodegenerative disease and has been exposed to the test substance with an expression level of the gene product in a target that has or is suspected of having a neurodegenerative disease and has not been exposed to the test substance and edaravone; and a step $\gamma$2 of selecting the test substance as a substance capable of treating or preventing a neurodegenerative disease when there is a change in the expression level of the gene product between the target that has been exposed to the test substance and the target that has not been exposed to the test substance and edaravone.

<26> The method according to <24> or <25>, wherein the step $\gamma$ includes: a step $\gamma$3 of comparing an expression level of the gene product in a target that has or is suspected of having a neurodegenerative disease and has been exposed to a test substance with an expression level of the gene product in a normal target that has not been exposed to a test substance and edaravone; and a step $\gamma$4 of selecting the test substance as a substance capable of treating or preventing a neurodegenerative disease when there is no change in the expression level of the gene product between a target that has been exposed to the test substance and a target that has not been exposed to the test substance but has been exposed to edaravone.

<27> The method according to <25> or <26>, wherein the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK and MAST1, and the step $\gamma$2 is a step of selecting the test substance as a substance capable of treating or preventing a neurodegenerative disease when the expression level of the gene product in a target that has been exposed to the test substance is lower than the expression level of the gene product in a target that has not been exposed to the test substance and edaravone.

<28> The method according to any one of <25> - <27>, wherein the gene product is a gene product of FAIM2, and the step $\gamma$2 is a step of selecting the test substance as a substance capable of treating or preventing a neurodegenerative disease when the expression level of the gene product in a target that has been exposed to the test substance is higher than the expression level of the gene product in a target that has not been exposed to the test substance and edaravone.

<29> The method according to any one of <24> - <28>, wherein the step $\gamma$ includes: a step $\gamma$5 of comparing an expression level of the gene product in a target that has or is suspected of having a neurodegenerative disease and has been exposed to a test substance with an expression level of the gene product in a target that has or is suspected of having a neurodegenerative disease and has not been exposed to a test substance but has been exposed to edaravone; and a step $\gamma$6 of evaluating that the test substance has a high possibility of being a substance capable of treating or preventing a neurodegenerative disease when there is no change in the expression level of the gene product between a target that has been exposed to the test substance and a target that has not been exposed to the test substance but has been exposed to edaravone.

<30> The method according to any one of <24> - <29>, wherein the step $\gamma$ includes: a step $\gamma$5 of comparing an expression level of the gene product in a target that has or is suspected of having a neurodegenerative disease and has been exposed to a test substance with an expression level of the gene product in a target that has or is suspected of having a neurodegenerative disease and has not been exposed to a test substance but has been exposed to edaravone; and a step $\gamma$7 of evaluating that the test substance has a low possibility of being a substance capable of treating or preventing a neurodegenerative disease when there is a change in the expression level of the gene product between a target that has been exposed to the test substance and a target that has not been exposed to the test substance but has been exposed to edaravone.

<31> The method according to any one of <24> - <30>, wherein the neurodegenerative disease is amyotrophic lateral sclerosis (ALS).

<32> The method according to any one of <24> - <31>, wherein the target is a human or a sample derived from a human.

<33> A biomarker for diagnosing a neurodegenerative disease or for diagnosing responsiveness to edaravone, the biomarker comprising a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2, preferably one or more genes selected from KAZALD1, SBK1, UBE2L6, NTM, DCTD, ASF1B and FCSK.

<34> A detection kit comprising a detection reagent capable of specifically detecting gene products of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2, for detecting one or more of the gene products as biomarkers for diagnosing a neurodegenerative disease or for diagnosing responsiveness to edaravone.

<35> A method for treating a neurodegenerative disease, the method comprising a step of administering the composition according to any one of <1> - <9> to a patient with a neurodegenerative disease.

<36> Use of a substance for causing a change in expression level of a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1, and FAIM2, for manufacturing a composition for prevention or treatment of a neurodegenerative disease.

<37> A method for suppressing occurrence or progression of cellular damage, the method comprising a step of causing a change in expression level of a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2 in cells.

<38> Edaravone or an edaravone-containing pharmaceutical composition for use in treatment or prevention of a neurodegenerative disease, such as ALS, by causing a change in expression level of a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1, and FAIM2.

<39> The composition, method, biomarker, detection kit, or use according to any one of <1> to <37>, wherein the gene is one, any combination of two or more, or all selected from KAZALD1, SBK1, UBE2L6, ALPL, NTM, PTTG1, ITGB4, HAUS4, DCTD, MT2A, ASF1B, FCSK, MAST1 and FAIM2, preferably, the gene is one, any combination of two or more, or all selected from KAZALD1, SBK1, UBE2L6, NTM, HAUS4, DCTD, ASF1B, FCSK and FAIM2, more preferably, the gene is one, any combination of two or more, or all selected from KAZALD1, SBK1, DCTD and FCSK, even more preferably, the gene is one, any combination of two, or all selected from SBK1, DCTD and FCSK, and most preferably, the gene is one or two selected from DCTD and FCSK.

[Examples]

In the following, the present invention is further described in detail based on examples. However, the scope of the present invention is not limited to such examples.

[Example 1: Preparation of disease model cells]

(1): Cell culture and differentiation

As a target, a 1464R adult rat neural stem cell line (hereinafter also referred to as the "1464R cell line" or "undifferentiated cell line") was used. The 1464R cell line was established using a method described in Neuropathology 2014, 34, 83-98.

The cell line was cultured in a non-adherent state in maintenance medium using a 10 cm dish treated with poly-2-hydroxyethyl methacrylate. Culture conditions were 37 °C in a 5% $CO_2$ environment, with subculturing performed twice a week.

The maintenance medium was prepared to contain the following components at their final concentrations in a Neurobasal Plus medium (manufactured by Thermo Fisher Scientific).

Typical neurosphere-forming 1464R cells were isolated. The isolated 1464R cells were dispersed and maintained in culture.

<Additives in the maintenance medium>

- 2 mM L-Glutamine (manufactured by Thermo Fisher Scientific)
- 2% v/v B-27 Supplement (manufactured by Thermo Fisher Scientific)
- 10 ng/mL Fibroblast Growth Factor (FGF)-2 (manufactured by Sigma)
- 10 ng/mL Epidermal Growth Factor (EGF) (manufactured by Sigma)
- 50 units/mL Penicillin and 50 μg/mL Streptomycin (manufactured by Thermo Fisher Scientific)

Next, using a differentiation medium shown below, the 1464R cell line was cultured in an adherent state on a poly-L-lysine treated dish. Culture conditions for cell differentiation were 37 °C, 5% $CO_2$, and 4 days. Through this step, the 1464R cell line was differentiated into neuronal cells (neurons). Further, the 1464R cell line partly differentiated into glial cells. In the following description, the differentiated 1464R cell line is also collectively referred to as the "differentiated cell line."

The differentiation medium was prepared to contain the following components at their final concentrations in an F-12 medium (manufactured by Thermo Fisher Scientific).

<Additives in the differentiation medium>

- 5 v/v% Fetal Bovine Serum (manufactured by Moregate)
- 0.5 v/v% N-2 supplement (manufactured by Thermo Fisher Scientific)
- 1 v/v% B-27 supplement (manufactured by Thermo Fisher Scientific)
- 1 μmol/L ATRA (manufactured by Sigma)
- 50 units/mL Penicillin and 50 μg/mL Streptomycin (manufactured by Thermo Fisher Scientific)

(2): Forced expression of TDP-43 protein

Subsequently, a neurodegenerative disease in vitro model was prepared by forcibly and excessively expressing TDP-43 protein in the differentiated cell line according to the following method.

After obtaining the differentiated cell line using the method described in (1), the differentiation medium was removed and replaced with infection medium. The composition of the infection medium was prepared to contain the following components at their final concentrations in F-12 medium (manufactured by Thermo Fisher Scientific) without an antioxidant and serum: The culture conditions were maintained at 37 °C in a 5% $CO_2$ environment.

<Additives in the infection medium>

- 0.5 v/v% N-2 supplement (manufactured by Thermo Fisher Scientific)
- 1 μmol/L ATRA (manufactured by Sigma)
- 50 units/mL Penicillin and 50 μg/mL Streptomycin (manufactured by Thermo Fisher Scientific)

Separately, the following recombinant adenovirus vectors (a) and (b) were prepared according to a method described in Neuropathology 2014, 34, 83-98.

(a) Wild-type: Recombinant adenovirus vector containing cDNA encoding full-length human wild-type TDP-43 labeled with DsRed (AxDsRhWTTDP43 strain; RIKEN DNA Bank Japan; #RDB15499)

(b) Mutant: Recombinant adenovirus vector containing cDNA encoding the C-terminal fragment of TDP-43 (amino

acid sequence numbers 208-414) labeled with DsRed (AxDsRhWTTDP43 strain; RIKEN DNA Bank Japan; #RDB 15500)

Using both vectors (a) and (b), the differentiated cell line in the infection medium was infected with adenovirus at a multiplicity of infection of 50, and cultured for 24 hours. As a result, neurons derived from the 1464R cell line, in which both wild-type and mutant TDP-43 were forcibly expressed intracellularly (hereinafter, also referred to as disease model cells), were obtained.

These disease model cells serve as an in vitro model of a neurodegenerative disease, in which TDP-43 expression localization and the presence of mutant TDP-43 protein may be involved. An example of such a neurodegenerative disease is ALS.

[Example 2: Evaluation of cell protection by microscopic observation]

Whether or not cellular damage is suppressed by edaravone (hereinafter also referred to as Eda) was evaluated using the disease model cells prepared using the method of Example 1.

Using a 96-well plate treated with poly-L-lysine, cells were seeded at a concentration of $8\times10^4$ cells/well, cultured and differentiated under the conditions of (1) in Example 1. After that, using the method of (2) in Example 1, TDP-43 was forcibly expressed in differentiated cells to obtain disease model cells.

For the disease model cells, as an Eda-free group, a group exposed to EA (final concentration 20 μmol/L) for 24 hours was prepared (See Fig. 1). Separately, as an Eda-containing group, a group exposed to Eda (final concentration 200 μmol/L) for 24 hours and then exposed to EA (final concentration 20 μmol/L) for 24 hours was prepared (See Fig. 2). Then, cell morphology in each group was observed using a fluorescence microscope.

Tubulin βIII (represented by TuJ1 in the figure), an indicator of neuronal differentiation, was fluorescently stained by fixing cells after 24 hours of EA exposure with 4% paraformaldehyde, treating them with TuJ1 antibody overnight at 4 °C, and then treating them with fluorescent antibody for 15 minutes at room temperature. Cell nuclei were fluorescently stained using Hoechst 33342 using a conventional method. The results are shown in Figs. 1 and 2.

As shown in Fig. 1, in the Eda-free group, it was confirmed that the neuron-specific protrusion shape observed by the localization of tubulin βIII had disappeared, indicating cellular damage had occurred.

On the other hand, as shown in Fig. 2, in the Eda-containing group, it was confirmed that the neuron-specific protrusion shape observed by the localization of tubulin βIII was sufficiently maintained, indicating a cell-protective effect. The localization of TDP-43 confirmed by DsRed, and the localization of cell nuclei were also confirmed to be present within the cells.

[Examples 3-1 to 3-6, Comparative Example 1, and Reference Example 1: Evaluation of cell protection using a chromogenic substrate as an indicator]

Disease model cells prepared using the method of Example 1 were used to prepare the following groups: Eda-containing groups (Examples 3-1 to 3-6): the cells were exposed to Eda (final concentration 1-200 μmol/L) for 24 hours, followed by exposure to EA (final concentration 20 μmol/L) for 24 hours; Eda-free group (Comparative Example 1): the cells were exposed to EA (final concentration 20 μmol/L) for 24 hours without prior exposure to Eda; and unexposed group (Reference Example 1): the cells were not exposed to either Eda or EA.

After that, a cell toxicity measurement kit (Cell Counting Kit-8, manufactured by DOJINDO) was used to expose the cells in each group according to a product protocol, and an amount of chromogenic substrate produced was measured by absorbance at 450 nm.

With this kit, the more viable cells present, the more chromogenic substrate is produced, resulting in a higher absorbance value at 450 nm. Therefore, a higher absorbance at 450 nm indicates a greater number of viable cells. The results are shown in Fig. 3.

The data shown in Fig. 3 are all expressed as mean ± standard error of the mean (SEM, n = 4 for each). In Fig. 3, the symbol "##" indicates a statistically significant difference of $p < 0.01$ compared to Comparative Example 1 (determined by Student t-test). In Fig. 3, the symbol "*" indicates a statistically significant difference of $p < 0.05$ compared to Comparative Example 1, and the symbol "**" indicates a statistically significant difference of $p < 0.01$ compared to Comparative Example 1 (both determined by Williams' multiple comparison test).

As shown in Fig. 3, in the Eda-containing groups (Examples 3-1 to 3-6), compared to the Eda-unexposed group (Comparative Example 1), the absorbance at 450 nm increased in an Eda concentration-dependent manner, confirming a higher number of viable cells. This indicates that cellular damage was suppressed in the presence of Eda. Further, the present inventor confirmed that similar results to those shown in Fig. 3 were obtained even when the disease model cells prepared using the method of Example 1 were simultaneously exposed to both Eda and EA for 24 hours.

[Example 4: Exploration and evaluation of biomarkers]

RNA-seq analysis was performed using the disease model cells prepared using the method of Example 1 to identify biomarkers that could serve as indicators for disease progression, evaluation of treatment efficacy, and/or selection of candidate compounds.

In the present evaluation, a 6-well plate treated with poly-L-lysine was used, cells were seeded at a density of $1\times10^6$ cells per well, cultured under the conditions of (1) of Example 1, and differentiated cells were used.

In the following, experimental groups (1) to (4) of the present example are shown along with Fig. 4.

(1) Group 1 (control group; represented as "Group 1" in Fig. 4 and Figs. 7-11): After preparing the differentiated cell line using the method of (1) of Example 1, the cells were further cultured in Eda and EA-free differentiation medium for 48 hours. This experimental group does not forcibly express TDP-43 and simulates healthy animals, including humans.

(2) Group 2 (Eda-only exposed group; represented as 'Group 2' in Fig. 4 and Figs. 7-11): After preparing the differentiated cell line using the method of (1) of Example 1, the cells were cultured in differentiation medium containing Eda (final concentration 50 μmol/L) but free of EA for 48 hours. This experimental group does not forcibly express TDP-43 and simulates conditions in which healthy animals, including humans, are exposed to Eda.

(3) Group 3 (TDP-43 + EA exposed group; represented as 'Group 3' in Fig. 4 and Figs. 7-11): After preparing the disease model cells expressing TDP-43, the cells were cultured in differentiation medium free of Eda but containing EA (final concentration 20 μ mol/L) for 24 hours. This experimental group forcibly expresses TDP-43 and simulates conditions where the target has a neurodegenerative disease and remains untreated.

(4) Group 4 (TDP-43 + EA + Eda exposed group; represented as 'Group 4' in Fig. 4 and Figs. 7-11): After preparing the disease model cells expressing TDP-43, the cells were cultured in differentiation medium containing Eda (final concentration 50 μmol/L) but free of EA for 24 hours. After that, EA (final concentration 20 μmol/L) was further added, and the cells were further cultured for an additional 24 hours. This experimental group simulates conditions where the target with a neurodegenerative disease is administered or exposed to Eda.

After the culture was completed, cell pellets containing $1\times10^6$ or more cells were obtained from the experimental groups (1) - (4). Total RNA was extracted from these cell pellets using NucleoSpin RNA (manufactured by Macherey-Nagel) according to a product protocol.

After that, using TapeStation and BioanalizerRNA6000 Nano Chip (manufactured by Agilent), it was confirmed that all of the extracted RNAs were of high quality, with an RNA concentration of 2 ng/μL or more and a total RNA amount of 50 ng or more.

Using the extracted total RNA (1.0 ng) as a template, double-stranded cDNA was synthesized using the SMART method using the SMART-Seq v4 Ultra (registered trademark) Low Input RNA Kit for Sequencing (manufactured by Clontech) according to a product protocol.

After that, the synthesized double-stranded cDNA was amplified by performing 13 cycles of PCR according to a product protocol. Then, a PCR product was purified using a magnetic bead method using AMPureXP (manufactured by Beckman Coulter).

Using the Nextera XT DNA Library Prep Kit and Nextera XT Index Kit v2 (manufactured by Illumina), the obtained PCR product (double-stranded cDNA) was tagmented and both ends of a fragmented cDNA were ligated with adapter sequences according to a product protocol. After that, the ligated cDNA (0.2 ng) was amplified by 12 cycles of PCR using a primer provided in the kit according to a product protocol. Then, the PCR products of all samples were pooled into one library to avoid any adverse effects that may occur during sequencing.

The PCR products obtained from the experimental groups were processed in several steps as shown in Fig. 5.

<RNA sequencing (see Fig. 5)>

Next, using the NovaSeq6000 system (NovaSeq6000 S4 reagent kit, NovaSeq Xp4 lane kit, and NovaSeq control software (version 1.6.0); all manufactured by Illumina), sequences of the cDNA library were determined according to a product protocol.

Base calls and quality scores were calculated using real-time analysis (version 3.4.4; manufactured by Illumina) according to cycle-based call files. The base call files were converted to FASTQ files using bcl2fastq2 software (version 2.20, manufactured by Illumina).

<Alignment - Expression Analysis (see Fig. 5)>

Next, using the DRAGEN Bio-IT platform software (version 3.6.3, manufactured by Illumina) and referring to the rat genome assembly (version 6.0, top-level) and transcript annotation (release 101) obtained from the Ensembl database, types and abundance of genes were identified from the 150-base sequence information contained in the FASTQ files mentioned above.

An RNA expression level (TPM) of a transcription product i measured from the cDNA library of the experimental groups was calculated based on alignment to the rat genome and transcription product annotation from the Ensembl database, according to the following equation (A).

In the following equation (A), "m_i" represents the number of reads for the transcription product i, and "l_i" represents the base length of the transcription product i.

$$TPM_i = 10^6 \times (m_i / l_i) / (\Sigma_i[(m_i / l_i)]) \ldots \quad (A)$$

Differential expression analysis was performed using DESeq2 (a package for two-group comparison) in the statistical analysis software R (version 3.6.0) to calculate a log2 ratio of RNA expression levels and a significance test. The log2 ratio is a value expressed as "log2 ([expression level of a first measurement target] / [expression level of a second measurement target])."

The significance test was performed based on a p-value calculated by Wald test with multiple testing correction based on the Benjamini-Hochberg method (BH method).

In the present analysis, a gene with an absolute log2 ratio of 1 or more (expression change ratio of 2.0 times or more or 0.50 times or less) and a p-value of 0.01 or less from the Wald test between any two groups was identified as a "gene with a change in expression level" (differentially expressed gene; DEG). DEGs include both upregulated genes and downregulated genes.

<Gene Ontology Analysis (see Fig. 5)>

Gene ontology analysis was performed by associating DEGs with ontology terms obtained from the BioMart database. These associations were performed by considering an ontology term with a p-value of 0.05 or less in the hypergeometric distribution test with multiple testing correction based on the BH method as significant. Classification of gene ontology related to DEGs was performed focusing on biological function, structural component, and molecular function, and significant ontology terms were extracted.

<Expression regulatory pathway analysis (see Fig. 5)>

Expression regulatory pathway analysis of DEGs was performed using software (Ingenuity Pathway Analysis, manufactured by QIAGEN). Association between expression pathways defined in the software and DEGs was performed by considering those with a p-value of 0.05 or less calculated by Fisher's exact test with multiple testing correction based on the BH method as significant.

Analysis results in the present example are shown in Fig. 6.

Fig. 6 shows results of principal component analysis of expression changes in RNA-seq data obtained from the experimental groups (N = 3 for each). The X-axis represents a first principal component, and the Y-axis represents a second principal component, with their respective contribution rates expressed as percentages.

The principal component analysis was performed by excluding genes with a TPM variance value of 1 or less across all samples.

As shown in Fig. 6, the RNA-seq data of samples from the same experimental group were all close to each other, indicating good quality of the RNA-seq data.

Comparing the experimental groups, it was found that Groups 3 and 4 had clearly different gene expression profiles compared to Group 1.

Further, it was found that Groups 3 and 4 had distinctly different gene expression profiles, despite both expressing TDP-43.

On the other hand, it was found that Groups 1 and 2 had similar gene expression profiles, regardless of the presence or absence of Eda exposure.

Table 1 below shows the results of identifying differentially expressed genes (DEGs) between the experimental groups based on the gene expression levels of the groups. In Table 1, "xxx/yyy" (where xxx and yyy independently represent any integers) indicates that the number of upregulated genes is xxx and the number of downregulated genes is yyy.

[Table 1]

| Group | Group 1 | Group 2 | Group 3 | Group 4 |
|---|---|---|---|---|
| Control (Group 1) | - | 0/0 | 853/467 | 1,654/1,095 |
| Eda (Group 2) | - | - | 876/478 | 1,661/1,130 |
| TDP43+EA (Group 3) | - | - | - | 169/260 |
| TDP43+EA+Eda (Group 4) | - | - | - | - |

From the differentially expressed genes in the experimental groups, genes meeting either criterion a or criterion b below were identified, and their expression change rates are shown in Figs. 7 to 11.

- Criterion a: Significantly upregulated in Group 3 compared to Group 1, and significantly downregulated in Group 4 compared to Group 3.
- Criterion b: Significantly downregulated in Group 3 compared to Group 1, and significantly upregulated in Group 4 compared to Group 3.

A gene that meets any of the above criteria means that its expression level changes with the onset of a disease (corresponding to Group 3) compared to that in a healthy state (corresponding to Group 1), and that its expression level returns to a level equivalent to or close to normal by treatment of the disease (corresponding to Group 4).

Therefore, the expression levels of these genes can serve as useful biomarkers, for example, by using changes in the expression levels as indicators, for: evaluating or predicting the possibility of the presence or absence of a disease and a degree of progression of the disease; evaluating or predicting the possibility of treatment responsiveness; selecting appropriate specimens for use as disease models; or selecting candidate compounds during drug discovery.

The genes shown in Figs. 7 to 11 are all rat genes that meet the above criteria and have human homologues.

As an example, Alpl (rat ortholog of human ALPL) shown in Fig. 8 (a) is a gene that encodes alkaline phosphatase. Alkaline phosphatase is an enzyme present in extracellular fluids such as blood, and thus, is advantageous, for example, for evaluating disease progression or treatment effectiveness or assisting in such predictions in a minimally invasive manner by collecting extracellular fluids such as blood.

Tables 2 and 3 below show the results of gene ontology analysis, as well as the differentially expressed genes, their expression change amounts (TPM), expression change log2 ratios, and p-values.

Further, Table 4 below shows the expression change log2 ratios and p-values for other differentially expressed genes, which are rat genes that meet the criteria a and b described above. All the genes listed in Table 4 are included in the specific genes of the present disclosure.

In Table 4, "Upregulated genes" are examples of genes whose expression levels are decreased by the presence of disease and increased by the presence of disease and edaravone. These genes are included in the gene group (B) described above.

In Table 4, "Downregulated genes" are examples of genes whose expression levels are increased by the presence of disease and decreased by the presence of disease and edaravone. These genes are included in the gene group (A) described above.

For convenience of description, the results for the Mt2A and Alpl genes listed in Tables 2 and 3 are re-presented in Table 4. The present inventor has performed quantitative PCR for Mt2A and Alpl and confirmed that expression profiles similar to those based on RNA-seq were obtained.

[Table 2]

<DEG> Group3 :Up / Group 4 :Down

| Gene Name | Description | Ontology | | TPM | | | | p value | log 2 ratio | p value | log 2 ratio |
|---|---|---|---|---|---|---|---|---|---|---|---|
| | | Biological.Process.Name | Molecular.Function.Name | Group 1 | Group 2 | Group 3 | Group 4 | Group 3 vs Group 1 | Group 3 / Group 1 | Group 4 vs Group 3 | Group 4 / Group 3 |
| Kazald1 | Kazal-type serine peptidase inhibitor domain 1 [Source:RGD Symbol;Acc:1310698] | regulation of cell growth // extracellular matrix organization | insulin-like growth factor binding // protein binding | 1.4317 | 1.0836 | 2.4495 | 1.2640 | 0.0008126214 | 1.2690256444 | 0.0010879349 | -1.1087202659 |
| Sbk1 | SH3 domain binding kinase 1 [Source:RGD Symbol;Acc:628713] | protein phosphorylation // phosphorylation // peptidyl-serine phosphorylation // brain development // peptidyl-threonine phosphorylation | nucleotide binding // protein kinase activity // protein serine/threonine kinase activity // ATP binding // kinase activity // transferase activity | 0.1665 | 0.5392 | 0.8017 | 0.0000 | 0.0076941548 | 1.4596792259 | 0.0000003849 | -1.6282275773 |
| Scn2a | sodium voltage-gated channel alpha subunit 2 [Source:RGD Symbol;Acc:3632] | ion transport // ion transmembrane transport // sodium ion transport // transmembrane transport // regulation of ion transmembrane transport // sodium ion transmembrane transport // cation transmembrane transport // nervous system development // memory // intrinsic apoptotic signaling pathway in response to osmotic stress // neuron apoptotic process | ion channel activity // voltage-gated ion channel activity // sodium channel activity // voltage-gated sodium channel activity // cation channel activity | 0.3581 | 0.5607 | 0.8328 | 0.2878 | 0.0050779118 | 1.1415965666 | 0.0006000647 | -1.2069923972 |
| Ube2l6 | ubiquitin-conjugating enzyme E2L 6 [Source:RGD Symbol;Acc:1307960] | ubiquitin-dependent protein catabolic process // protein ubiquitination // protein polyubiquitination // modification-dependent protein catabolic process // ISG15-protein conjugation | nucleotide binding // ATP binding // transferase activity // ubiquitin conjugating enzyme activity // ubiquitin binding // ubiquitin protein ligase binding // ISG15 transferase activity // ubiquitin-like protein transferase activity | 1.8012 | 2.1006 | 2.6385 | 1.3251 | 0.0019746140 | 1.0537975341 | 0.0029075887 | -1.0920725655 |
| Alpl | alkaline phosphatase, biomineralization associated [Source:RGD Symbol;Acc:2100] | dephosphorylation // response to lipopolysaccharide // response to glucocorticoid // endochondral ossification // cellular response to organic cyclic compound // response to antibiotic // response to vitamin D // cementum mineralization // developmental process involved in reproduction | hydrolase activity // catalytic activity // phosphatase activity // alkaline phosphatase activity // metal ion binding // pyrophosphatase activity | 1.4457 | 1.5309 | 2.4333 | 1.3164 | 0.0001392364 | 1.3442994880 | 0.0010568103 | -1.0975313910 |
| Ntm | neurotrimin [Source:RGD Symbol;Acc:620958] | - | - | 1.0515 | 1.1081 | 2.0269 | 0.9620 | 0.0003320477 | 1.3595785106 | 0.0011423348 | -1.1121754907 |
| Pttg1 | PTTG1 regulator of sister chromatid separation, securin [Source:RGD Symbol;Acc:68359] | negative regulation of cell population proliferation // negative regulation of endopeptidase activity // chromosome segregation // DNA repair // cellular response to DNA damage stimulus // cell division // transcription by RNA polymerase II // cell cycle // mitotic sister chromatid cohesion // regulation of cell growth // regulation of transcription by RNA polymerase II // homologous chromosome segregation // chromosome organization // negative regulation of mitotic sister chromatid separation | protein binding // DNA-binding transcription factor activity, RNA polymerase II-specific // cysteine-type endopeptidase inhibitor activity // ribosome binding // heat shock protein binding // SH3 domain binding | 1.5503 | 1.4190 | 2.6045 | 1.0105 | 0.0066050970 | 1.1990189219 | 0.0016092214 | -1.2591052930 |

[Table 3]

<DEG> Group3 :Up / Group 4 :Down

| Gene Name | Description | Ontology | | TPM | | | | p value | log 2 ratio | p value | log 2 ratio |
|---|---|---|---|---|---|---|---|---|---|---|---|
| | | Biological.Process.Name | Molecular.Function.Name | Group 1 | Group 2 | Group 3 | Group 4 | Group 3 vs Group 1 | Group 3 / Group 1 | Group 4 vs Group 3 | Group 4 / Group 3 |
| Itgb4 | integrin subunit beta 4 [Source:RGD Symbol;Acc:2928] | cell motility // hemidesmosome assembly // integrin-mediated signaling pathway // cell communication // cell adhesion // cell-matrix adhesion // autophagy // response to wounding // nail development // skin morphogenesis // mesodermal cell differentiation | protein binding // G protein-coupled receptor binding // insulin-like growth factor I binding // neuregulin binding | 1.7427 | 1.9611 | 2.8300 | 1.2417 | 0.0000000001 | 1.3949666803 | 0.0000000005 | -1.6036322777 |
| Haus4 | HAUS augmin-like complex, subunit 4 [Source:RGD Symbol;Acc:1305288] | centrosome cycle // spindle assembly | microtubule minus-end binding | 2.9144 | 2.9066 | 3.8677 | 2.6313 | 0.0000001849 | 1.1103292916 | 0.0000069443 | -1.0392806987 |
| Dctd | dCMP deaminase [Source:RGD Symbol;Acc:1359671] | pyrimidine nucleotide metabolic process | dCMP deaminase activity // metal ion binding // zinc ion binding // catalytic activity // hydrolase activity // identical protein binding | 1.6212 | 2.0805 | 2.5046 | 1.2658 | 0.0003910350 | 1.1154052633 | 0.0001849461 | -1.1577443798 |
| Mt2A | metallothionein 2A [Source:RGD Symbol;Acc:1592345] | response to bacterium // cellular response to drug // cellular zinc ion homeostasis // cellular response to erythropoietin // detoxification of copper ion // cellular response to cadmium ion // cellular response to copper ion // cellular response to zinc ion // nitric oxide mediated signal transduction // cellular response to interleukin-3 // astrocyte activation // negative regulation of growth | metal ion binding // zinc ion binding // drug binding // cadmium ion binding | 3.7248 | 3.7836 | 5.9682 | 3.7564 | 0.0000000059 | 2.1339362089 | 0.0000911021 | -1.4249781799 |
| Asf1b | anti-silencing function 1B histone chaperone [Source:RGD Symbol;Acc:1304918] | chromatin assembly or disassembly // nucleosome assembly // DNA replication-dependent nucleosome assembly // DNA replication-independent nucleosome assembly // blastocyst hatching | histone binding | 1.4821 | 1.8799 | 2.2951 | 0.9563 | 0.0079022897 | 1.0478306390 | 0.0002496304 | -1.3225463606 |
| Fcsk | fucose kinase [Source:RGD Symbol;Acc:1307000] | GDP-L-fucose salvage // carbohydrate phosphorylation // response to dopamine // phosphorylation | nucleotide binding // ATP binding // transferase activity // transferase activity, transferring phosphorus-containing groups // fucokinase activity // kinase activity | 2.2527 | 2.5696 | 3.1070 | 1.9393 | 0.0000000027 | 1.0816159987 | 0.0000047070 | -1.0472344181 |
| MAST1 | microtubule associated serine/threonine kinase 1 [Source:RGD Symbol;Acc:631372] | protein phosphorylation // phosphorylation // brain development // cytoskeleton organization // intracellular signal transduction // peptidyl-serine phosphorylation | nucleotide binding // protein kinase activity // protein serine/threonine kinase activity // ATP binding // kinase activity // transferase activity // metal ion binding // magnesium ion binding // microtubule binding // protein binding | 1.0395 | 0.7213 | 1.9228 | 0.9064 | 0.0000118126 | 1.3752092632 | 0.0031650078 | -1.0521818896 |

<DEG> Group3 :Down / Group 4 :Up

| Gene Name | Description | Ontology | | TPM | | | | p value | log 2 ratio | p value | log 2 ratio |
|---|---|---|---|---|---|---|---|---|---|---|---|
| | | Biological.Process.Name | Molecular.Function.Name | Group 1 | Group 2 | Group 3 | Group 4 | Group 3 vs Group 1 | Group 3 / Group 1 | Group 4 vs Group 3 | Group 4 / Group 3 |
| Faim2 | Fas apoptotic inhibitory molecule 2 (protein lifeguard 2-like LOC102551901) [Source:RGD Symbol;Acc:7636626] | - | - | 2.2911 | 2.2182 | 0.7312 | 1.7210 | 0.0000049024 | -1.9860314510 | 0.0017093350 | 1.2684526239 |

[Table 4]

| Gene Name | Description | Group 3 to Group 1 | | Group 4 to Group 3 | |
|---|---|---|---|---|---|
| | | log2 ratio | p values | log2 ratio | p values |
| **Upregulated genes**[a] | | | | | |
| Ho-1 | heme oxygenase 1 | 6.601 | 0.000E+00 | 1.755 | 4.712E-44 |
| Cebpb | CCAAT/enhancer binding protein beta | 2.930 | 4.358E-164 | 1.661 | 1.309E-28 |
| Plpp3 | phospholipid phosphatase 3 | 0.751 | 7.027E-04 | 1.307 | 3.287E-10 |
| Sqstm1 | sequestosome 1 | 1.366 | 1.227E-55 | 1.225 | 2.665E-26 |
| Pik3cb | phosphatidylinositol-4,5-bisphosphate 3-kinase, catalytic subunit beta | 0.879 | 8.668E-11 | 1.157 | 2.285E-18 |
| Txndc2 | thioredoxin domain containing 2 | 0.917 | 4.981E-02 | 1.153 | 5.690E-03 |
| Hspa8 | heat shock protein family A (Hsp70) member 8 | 1.931 | 1.714E-79 | 1.151 | 4.385E-13 |
| Hsph1 | heat shock protein family H (Hsp110) member 1 | 4.000 | 0.000E+00 | 1.027 | 2.124E-07 |
| Ptgds | prostaglandin D2 synthase | -1.115 | 1.478E-04 | 0.954 | 2.334E-03 |
| Nfe2l2 | nuclear factor, erythroid 2-like 2 | 1.898 | 1.063E-43 | 0.923 | 9.336E-11 |
| Plcg2 | phospholipase C, gamma 2 | -1.570 | 3.015E-11 | 0.881 | 4.049E-03 |
| **Downregulated genes**[b] | | | | | |
| Nefl | neurofilament light | 0.738 | 1.168E-01 | -1.778 | 5.902E-07 |
| Acsl6 | acyl-CoA synthetase long-chain family member 6 | -1.225 | 2.073E-06 | -1.582 | 1.693E-05 |
| Mt2A | metallothionein 2A | 2.134 | 5.863E-09 | -1.425 | 9.110E-05 |
| Mt1 | metallothionein 1 | 0.866 | 8.031E-06 | -1.392 | 3.600E-09 |
| Glrx | glutaredoxin | 0.726 | 3.319E-03 | -1.262 | 8.634E-06 |
| Alpl | alkaline phosphatase, biomineralization associated | 1.344 | 1.392E-04 | -1.098 | 1.057E-03 |
| Nup43 | nucleoporin 43 | 0.947 | 6.178E-05 | -0.888 | 1.606E-04 |

a) Up-regulated significantly (adjusted p-value ≤ 0.01 and/or log2 fold change ≥ 1) in Group 4 compared to Group 3
b) Down-regulated significantly (adjusted p-value ≤ 0.01 and/or log2 fold change ≤ -1) in Group 4 compared to Group 3

[Examples 5-1 and 5-2, Comparative Example 2, and Reference Examples 2 and 3: Evaluation of cell protection using a chromogenic substrate as an indicator (2)]

Using the differentiated cell line prepared using the method of Example 1 (indicated as "TDP-43 (-)" in Fig. 12), an Eda-unexposed group (Reference Example 2) and a 96-hour Eda-exposed group (final concentration 100 μmol/L) (Reference Example 3) were each prepared.

Further, using the disease model cells prepared using the method of Example 1 (indicated as "TDP-43 (+)" in Fig. 12), an Eda-unexposed group (Comparative Example 2) and 96-hour Eda-exposed groups (final concentration 50 or 100 μmol/L) (Examples 5-1 and 5-2) were each prepared.

All of these were performed without containing EA.

After that, similar to Example 3-1 and the like, a cell toxicity measurement kit was used to expose the cells in each group according to a product protocol, and an amount of chromogenic substrate produced was measured by absorbance at 450 nm. The results are shown in Fig. 12.

The data shown in Fig. 12 are all expressed as mean ± standard error of the mean (SEM, n = 4 for each). In Fig. 12, the symbol "##" indicates a statistically significant difference of $p < 0.01$ compared to Comparative Example 2. In Fig. 12, the symbol "**" indicates a statistically significant difference of $p < 0.05$ compared to Comparative Example 2. In Fig. 12, the symbol "$" indicates a statistically significant difference of $p<0.05$ compared to Reference Example 2 (all determined by Student t-test).

As shown in Fig. 12, in the TDP-43 presence and Eda-containing groups (Examples 5-1 to 5-2), a higher number of viable cells was confirmed compared to the TDP-43 presence and Eda-unexposed group (Comparative Example 2).

This indicates that even in the absence of EA, cellular damage occurred in the presence of TDP-43, and that Eda suppressed cellular damage caused by TDP-43.

[Example 6: Preparation of disease model cells (2)]

Human induced pluripotent stem (iPS) cells were induced from cells derived from an ALS patient and cells derived from a healthy individual using a conventional method. The cells derived from the ALS patient were used that are known to have an ALS risk mutation as a heterozygous mutation in the TARDBP gene. Each iPS cell was differentiated into a motor neuron (hereinafter, also simply referred to as a "neuron") using a conventional method. Neurons differentiated from iPS cells induced from cells derived from an ALS patient are simply referred to as "ALS patient-derived neurons," and neurons differentiated from iPS cells induced from cells derived from a healthy individual are simply referred to as "healthy individual-derived neurons." For long-term preservation of the cells, when necessary, the neurons were subjected to a freezing treatment 2 to 7 days after the differentiation treatment. Neurons that have been subjected to the freezing treatment are also referred to as "frozen neurons."

Subsequently, neurons were cultured using a 98-well culture plate (Corning, 3548) under the conditions shown below. Coating of the culture plate was performed in advance before cell seeding. Specifically, a 0.02% Poly-L-Ornithine solution (Sigma-Aldrich, P4957) was added to the wells of the culture plate, and left to stand for 2 hours in a 37 °C 5% $CO_2$ incubator. After that, the wells were washed, and a 20 mg/mL Laminin solution (Thermo Fisher Scientific, 23017015) was used and it was left to stand for a further 2 hours in a 37 °C 5% $CO_2$ incubator. After that, the frozen neurons were thawed using a conventional method, and the thawed neurons were seeded into the wells at 20,000 cells/well, and the neurons were cultured in the presence of a culture medium having the following composition.

The culture medium used was a mixture with the following composition.

- DMEM/F12 (Thermo Fisher Scientific, 21331-020): 50% v/v
- Neurobasal Medium (Thermo Fisher Scientific, 21103-049): 50% v/v
- Glutamax Supplement (Thermo Fisher Scientifc, 35050061): 1% v/v
- Penicillin-Streptomycin (10000 units/mL) (Thermo Fisher Scietific, 15140-148): 0.5% v/v
- Component N1 (Elixirgen): 3% v/v
- Component A (Elixirgen): 0.1% v/v
- Component D4 (Elixirgen): 0.1% v/v
- Component P (Elixirgen): 0.05% v/v

[Example 7: Evaluation of cellular damage by live cell imaging]

A treatment was performed in which the culture medium dissolved with a dimethyl sulfoxide (DMSO) solution of the evaluation compound (edaravone) (final concentration: edaravone 30 $\mu$M, DMSO 0.03% v/v) was brought into contact with the neurons after 7 days of cultivation, and occurrence of cellular damage was evaluated over time. Separately, a group without the evaluation compound in the culture medium (a group with the same concentration of DMSO added) was also prepared.

Evaluation of cellular damage was performed by live cell imaging using IncuCyte S3 (Sartorius) and Cytotox (Sartorius, 4846) according to an attached protocol. The neurite length per unit area (unit: $mm/mm^2$) and the number of dead cells (unit: count/image) were analyzed over time and used as evaluation indicators. Smaller values of neurite length and higher numbers of dead cells indicate greater cellular damage.

<Cellular damage evaluation (1) based on neurite length>

Fig. 13 shows the results of calculating the rate of change in neurite length for each experimental group from the start of the evaluation compound treatment to the time of analysis (24 hours), expressed as a ratio where the DMSO group without the evaluation compound is set to 100. The smaller the value on the vertical axis, the more the neurite length decreases over time, indicating that cellular damage has occurred. The experimental groups in Fig. 13 are as follows.

- ALS cells-DMSO: A group using ALS patient-derived neurons, with no edaravone in the culture medium
- ALS cells-edaravone: A group using ALS patient-derived neurons, with edaravone in the culture medium
- Healthy cells-DMSO: A group using healthy individual-derived neurons, with no edaravone in the culture medium
- Healthy cells-edaravone: A group using healthy individual-derived neurons, with edaravone in the culture medium

When the edaravone treatment was applied to both healthy individual-derived neurons and ALS patient-derived neurons, the neurite length in the ALS patient-derived neurons was significantly increased compared to the DMSO-treated group, indicating a protective effect on the neurites. In contrast, no significant change in neurite length was observed in the healthy individual-derived neurons.

<Cellular damage evaluation (2) based on the number of dead cells>

Fig. 14 shows the results of calculating the rate of change in cell death for each experimental group from the start of the evaluation compound treatment to the time of analysis (24 hours), expressed as a ratio where the DMSO-treated group without the evaluation compound is set to 100. The larger the value on the vertical axis, the greater the number of dead cells over time, indicating that cellular damage has occurred. The experimental groups in Fig. 14 are as follows.

- ALS cells-DMSO: A group using ALS patient-derived neurons, with no edaravone in the culture medium
- ALS cells-edaravone: A group using ALS patient-derived neurons, with edaravone in the culture medium
- Healthy cells-DMSO: A group using healthy individual-derived neurons, with no edaravone in the culture medium
- Healthy cells-edaravone: A group using healthy individual-derived neurons, with edaravone in the culture medium

When the edaravone treatment was applied to both healthy individual-derived neurons and ALS patient-derived neurons, the number of dead neurons in the ALS patient-derived neurons was significantly decreased compared to the DMSO-treated group, indicating a protective effect against neuronal cell death. In contrast, no significant change in the number of dead neurons was observed in the healthy individual-derived neurons.

[Example 8: TDP-43 localization evaluation]

Intracellular localization of TDP-43 polypeptides was evaluated by immunofluorescence staining of neurons. The cultured neurons were washed with phosphate-buffered saline (PBS) and fixed with 4% paraformaldehyde (PFA). After blocking with PBS containing 5% fetal bovine serum (FBS) and 0.1% Triton™-X, a primary antibody dilution containing anti-TDP-43 antibody and anti-β-III tubulin antibody was added and incubated overnight at 4 °C. The anti-β-III tubulin antibody was used to visualize the neuronal cell bodies. The next day, after washing with PBS, a secondary antibody dilution conjugated with Alexa dye was added and incubated for 1 hour at room temperature. After further nuclear staining, washing with PBS, and imaging under a fluorescence microscope were performed. The captured images were analyzed using Matlab (Mathworks), and fluorescence intensities of TDP-43 in a nuclear region and in a cytoplasmic region were respectively calculated. The results of the intracellular localization of the TDP-43 polypeptide are shown in Fig. 15. The experimental groups in Fig. 15 are as follows.

- ALS cells-DMSO: A group using ALS patient-derived neurons, with no edaravone in the culture medium
- ALS cells-edaravone: A group using ALS patient-derived neurons, with edaravone in the culture medium
- Healthy cells-DMSO: A group using healthy individual-derived neurons, with no edaravone in the culture medium
- Healthy cells-edaravone: A group using healthy individual-derived neurons, with edaravone in the culture medium

Regarding the intracellular localization of TDP-43 polypeptides, the nuclear and cytoplasmic TDP-43 fluorescence intensities and the cytoplasmic/nuclear TDP-43 intensity ratios in ALS cells and healthy cells are shown in Fig. 15. The values are presented as ratios with the DMSO group set to 100. When the edaravone treatment was applied to both healthy individual-derived neurons and ALS patient-derived neurons, the cytoplasmic TDP-43 intensity and the cytoplasmic/nuclear TDP-43 intensity ratio significantly decreased in ALS patient-derived neurons after 24 hours, indicating that the abnormal localization of TDP-43 observed in ALS pathology is suppressed. Further, such an effect of decreasing the cytoplasmic/nuclear TDP-43 intensity ratio was not observed in the healthy individual-derived neurons.

From the above results, it was newly demonstrated that edaravone, an already approved drug for ALS, has the effect of suppressing cellular damage in ALS patient-derived neurons and improving the abnormal intracellular localization of TDP-43. The abnormal localization of TDP-43 is a pathological condition commonly observed in a pathological condition of ALS, which is a heterogeneous disease, and demonstrating an effect in correcting the abnormal localization of TDP-43 indicates the possibility of showing efficacy in ALS patients in general. Further, without being limited to ALS, it can contribute to beneficial effects in the treatment or prevention or the like of diseases related to the abnormal localization of TDP-43. These are new findings that have become clear through the completion of the present invention, and the results indicate that edaravone can be a suitable therapeutic or preventive method for diseases associated with abnormal localization of TDP-43.

## Claims

1. A composition for causing a change in expression level of a gene product in a target, the composition comprising edaravone, wherein the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2.

2. The composition according to claim 1, used as a pharmaceutical containing edaravone as an active ingredient.

3. The composition according to claim 2, used for treating or preventing a neurodegenerative disease.

4. The composition according to claim 3, wherein the neurodegenerative disease is amyotrophic lateral sclerosis (ALS).

5. The composition according to any one of claims 1 - 4, wherein the target is a mammal or a sample derived from a mammal.

6. A method for evaluating responsiveness of a target to edaravone, the method comprising

    a step of evaluating whether or not there is a possibility that the target has responsiveness to edaravone based on a change in expression level of a gene product due to exposure of the target to edaravone, wherein
    the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2.

7. A method for evaluating a possibility of having a neurodegenerative disease of a target, the method comprising

    a step of evaluating whether or not there is a possibility that the target has a neurodegenerative disease based on a change in expression level of a gene product in the target, wherein
    the gene product is a gene product of one or more genes selected from KAZALD1, SBK1, UBE2L6, NTM, DCTD, ASF1B and FCSK.

8. A method for screening a substance capable of treating or preventing a neurodegenerative disease, the method comprising

    a step of selecting a test substance as a substance capable of treating or preventing a neurodegenerative disease based on a change in expression level of a gene product due to exposure of a target to the test substance, wherein
    the gene product is a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2.

9. A biomarker for diagnosing a neurodegenerative disease, the biomarker comprising a gene product of one or more genes selected from KAZALD1, SBK1, UBE2L6, NTM, DCTD, ASF1B and FCSK.

10. A biomarker for diagnosing edaravone responsiveness, the biomarker comprising a gene product of one or more genes selected from KAZALD1, SBK1, SCN2A, UBE2L6, ALPL, NTM, PTTG1, ITGB4, HAUS4, DCTD, MT2A, ASF1B, FCSK, MAST1 and FAIM2.

FIG. 1

AxDsR/
TDP43.WT
+
AxDsR/
TDP43.CTF
+
Ethacrynic acid
20μM
24hr

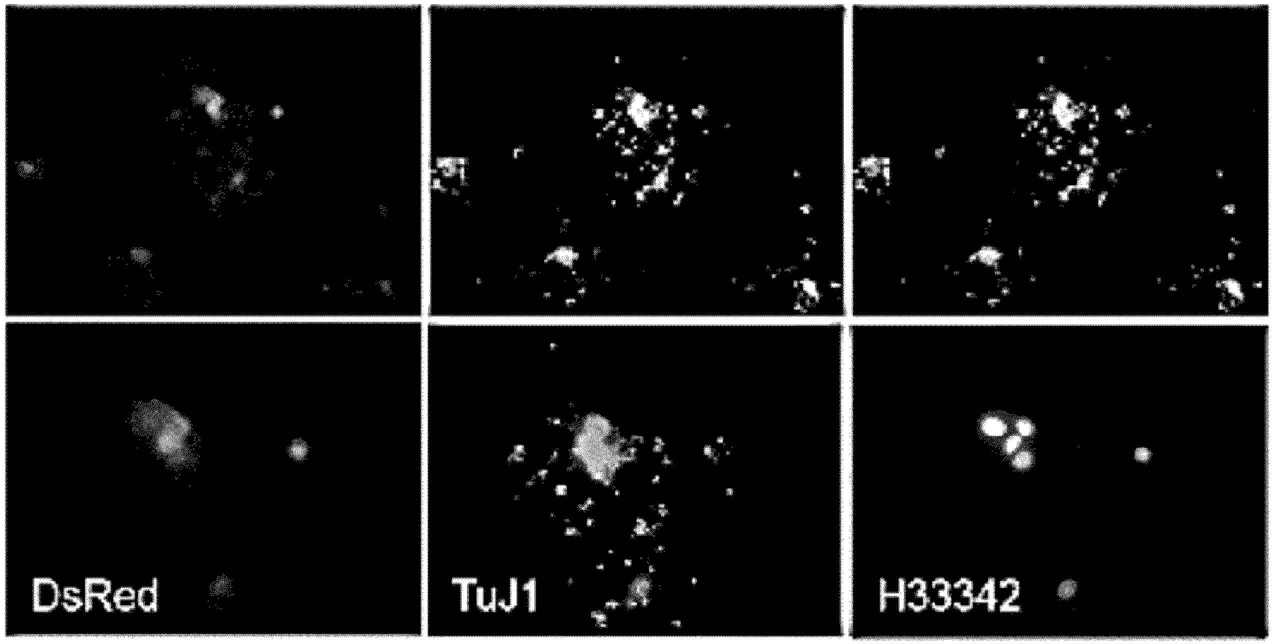

FIG. 2

AxDsR/
TDP43.WT
+
AxDsR/
TDP43.CTF
+
Ethacrynic acid
20μM
+
Edaravone
200μM
24hr

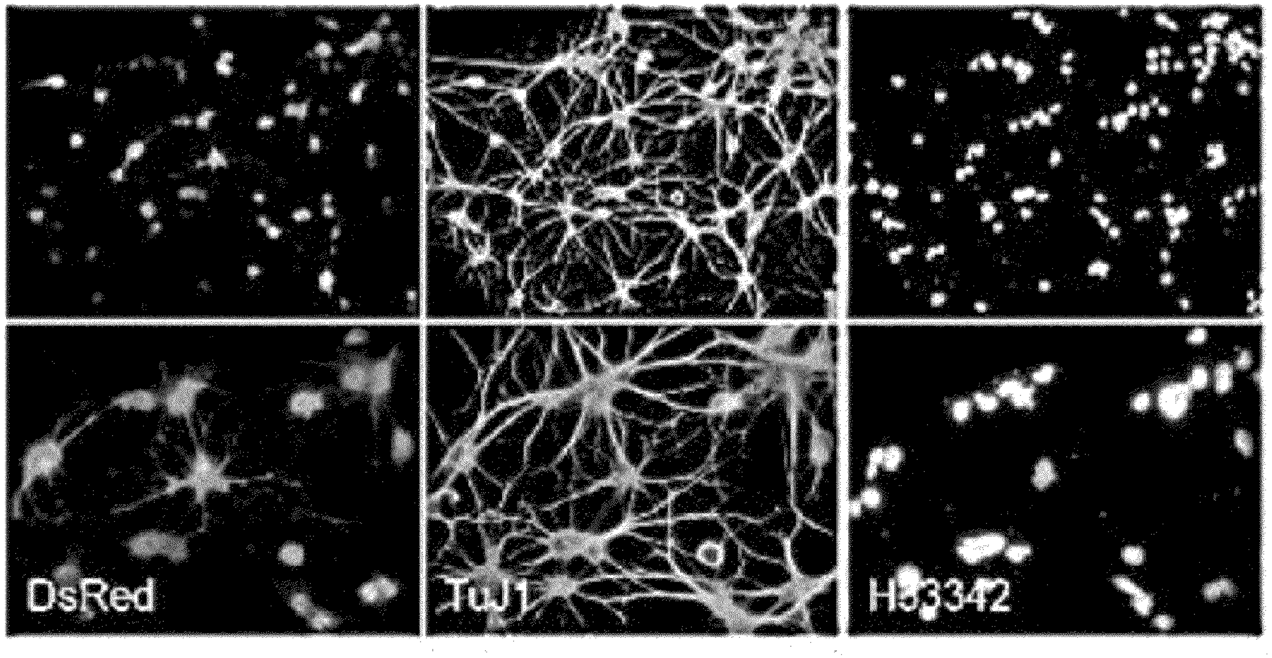

FIG. 3

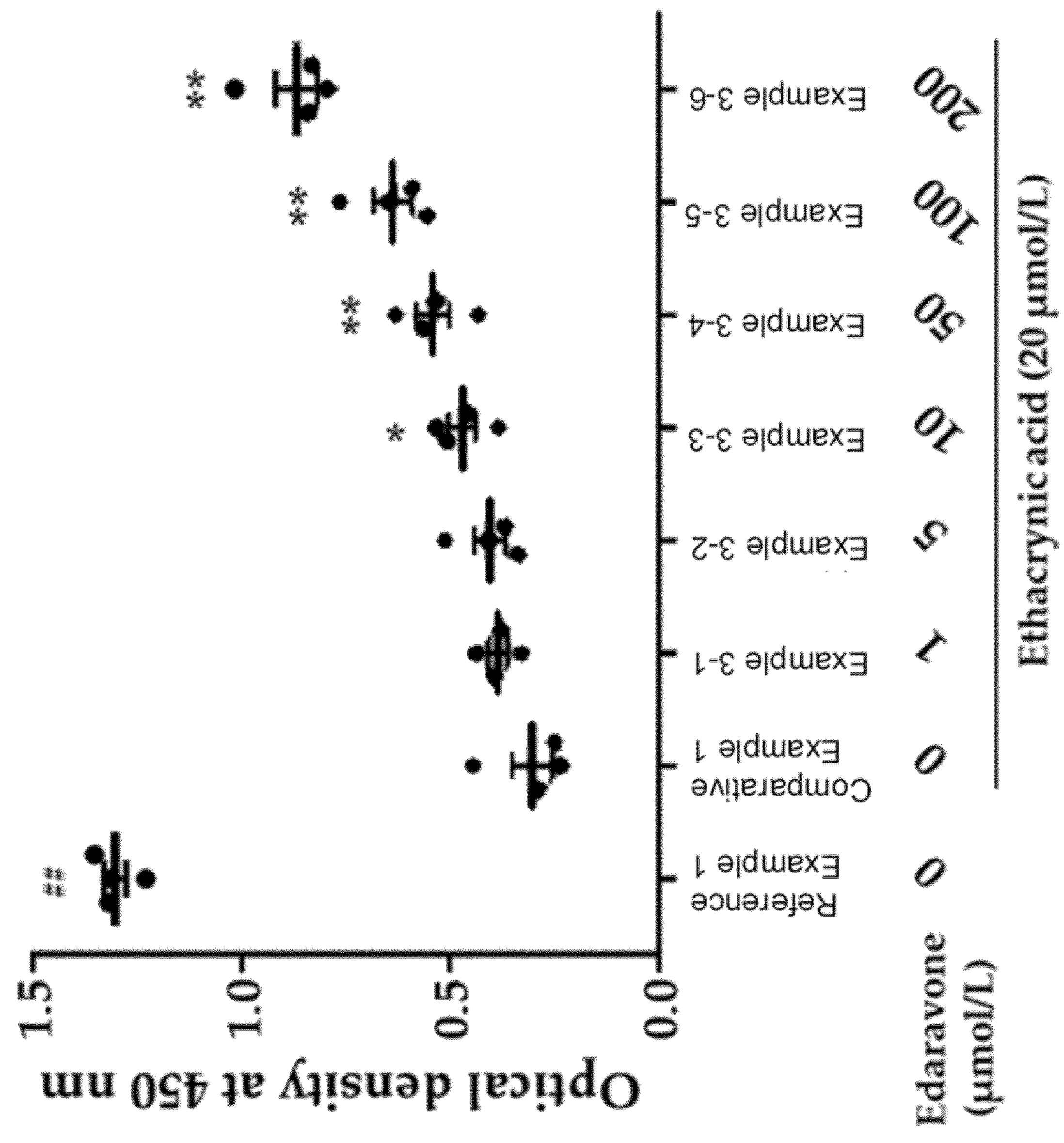
Optical density at 450 nm
1.5
1.0
0.5
0.0
##
*
**
**
**
Reference Example 1
Comparative Example 1
Example 3-1
Example 3-2
Example 3-3
Example 3-4
Example 3-5
Example 3-6
Edaravone (μmol/L)
0
0
1
5
10
50
100
200
Ethacrynicacid (20 μmol/L)

FIG. 4

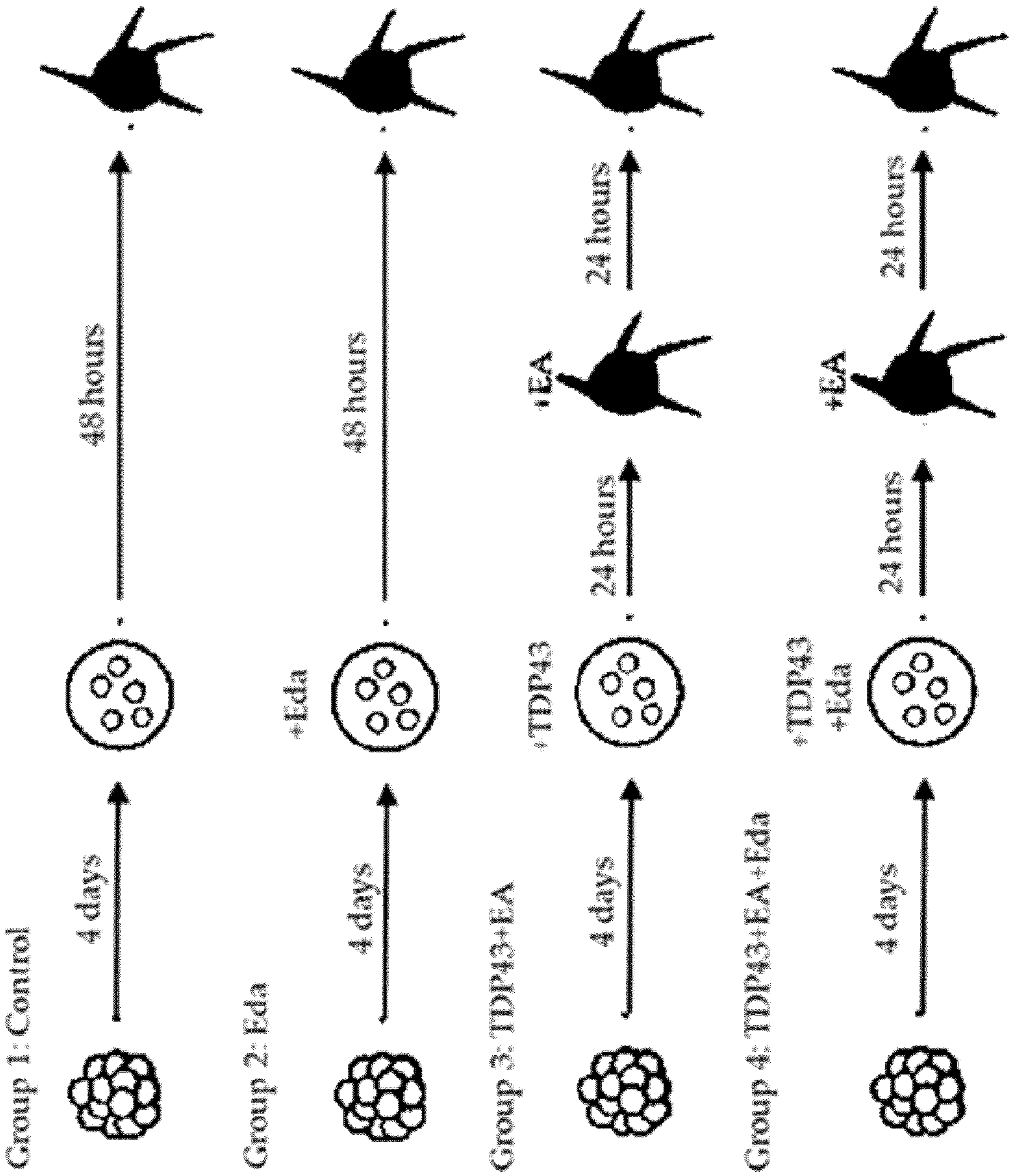
Group 1: Control
4 days
48 hours
Group 2: Eda
4 days
+Eda
48 hours
Group 3: TDP43+EA
4 days
+TDP43
24 hours
+EA
24 hours
Group 4: TDP43+EA+Eda
4 days
+TDP43
+Eda
24 hours
+EA
24 hours

FIG. 5

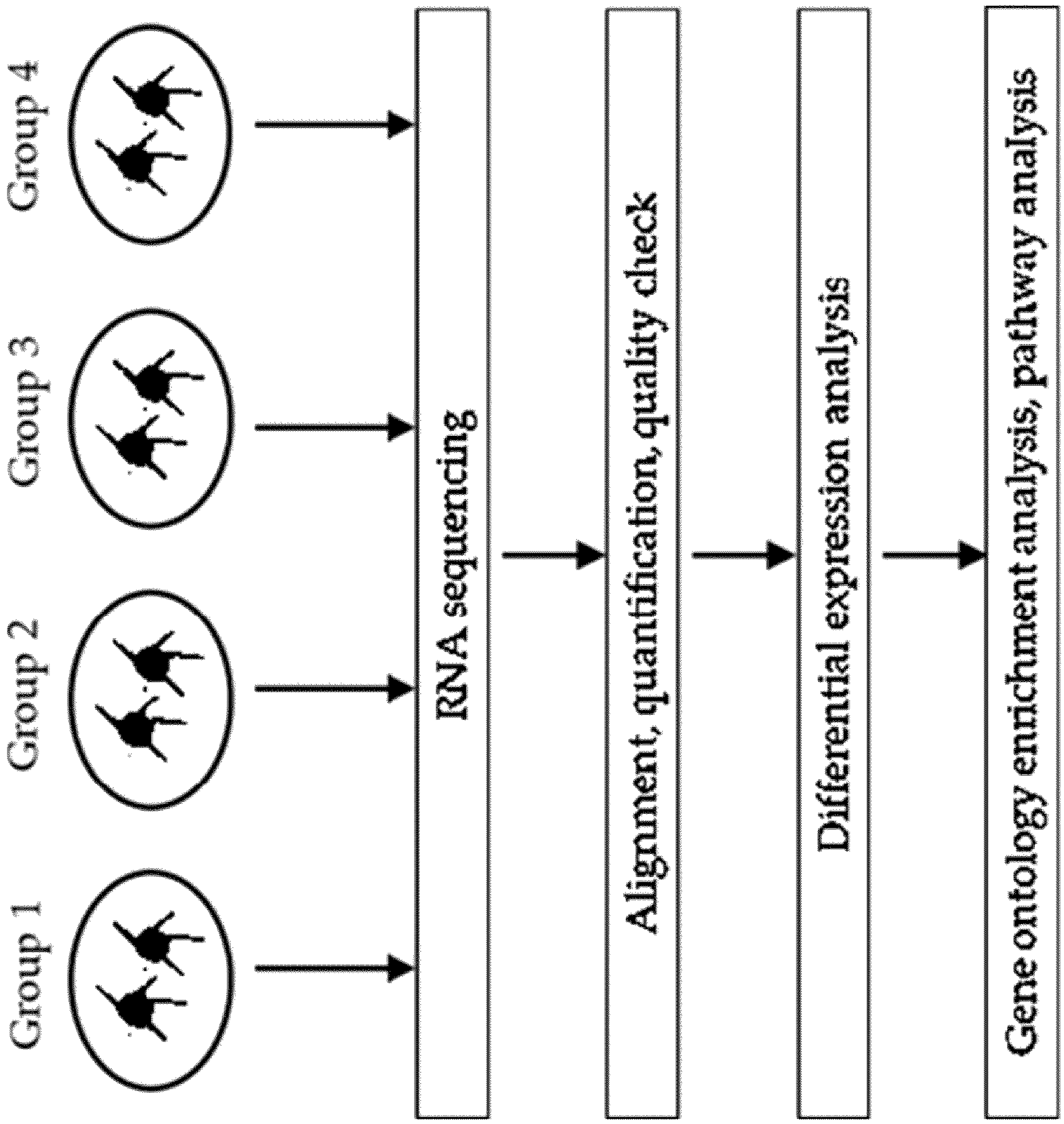
Group 1
Group 2
Group 3
Group 4
RNA sequencing
Alignment, quantification, quality check
Differential expression analysis
Gene ontology enrichment analysis, pathway analysis

FIG. 6

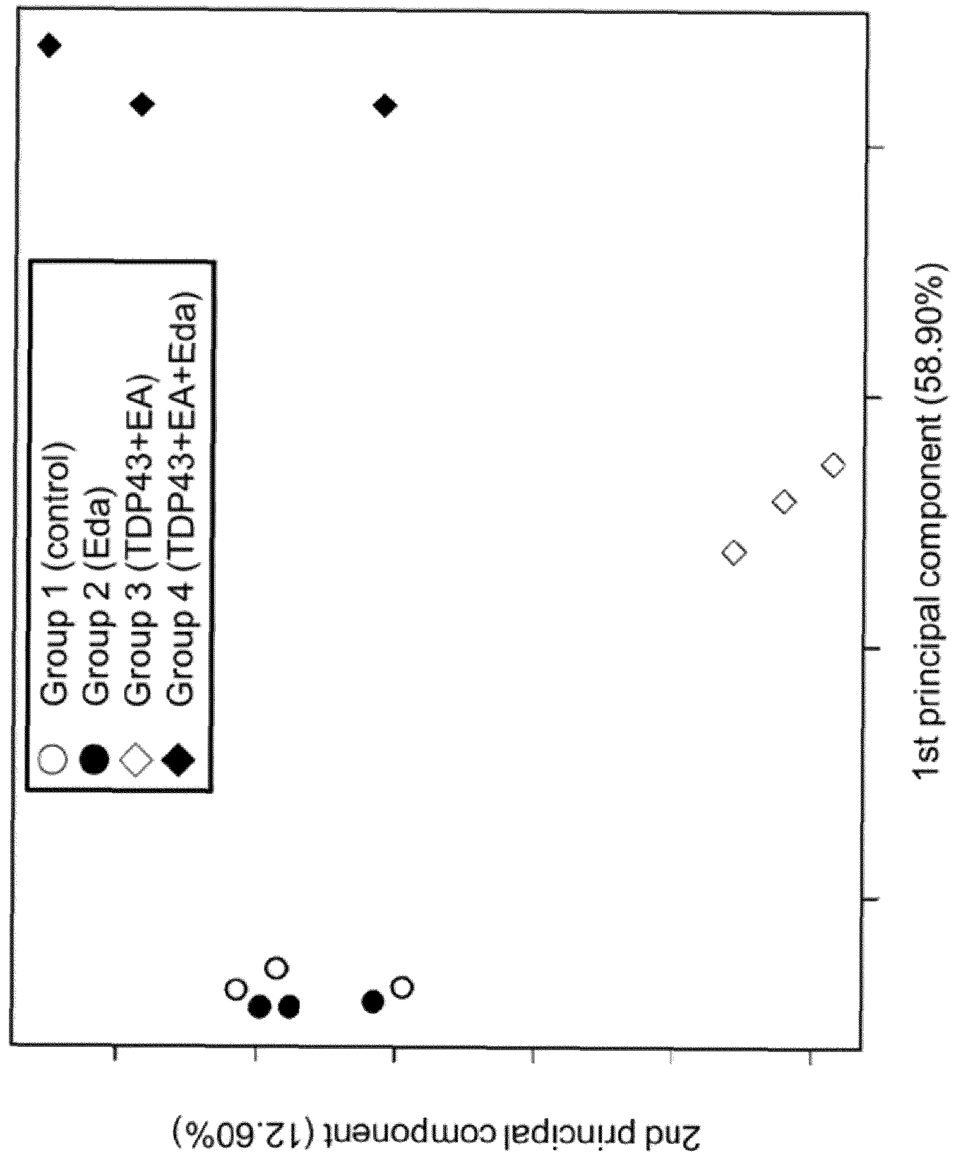
Group 1 (control)
Group 2 (Eda)
Group 3 (TDP43+EA)
Group 4 (TDP43+EA+Eda)
1st principal component (58.90%)
2nd principal component (12.60%)

Fig.7(a)

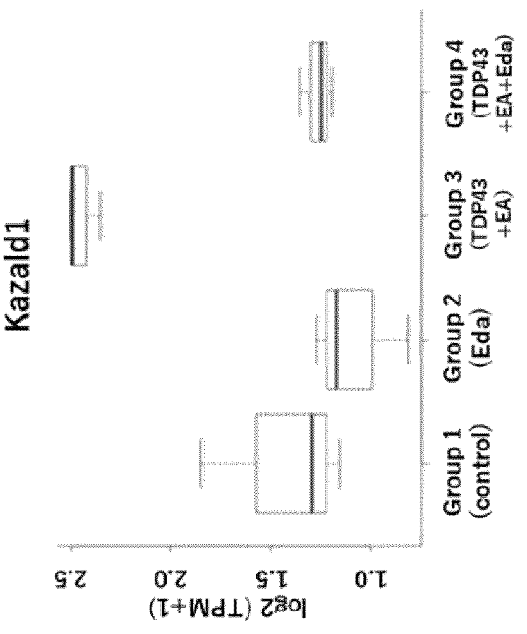


Fig.7(b)

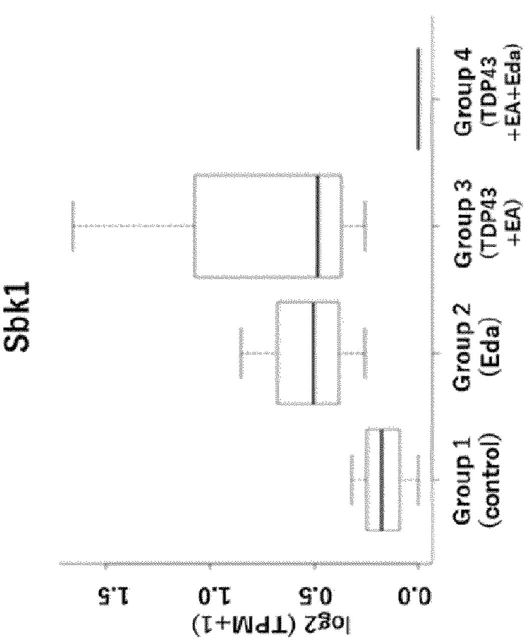


Fig.7(c)

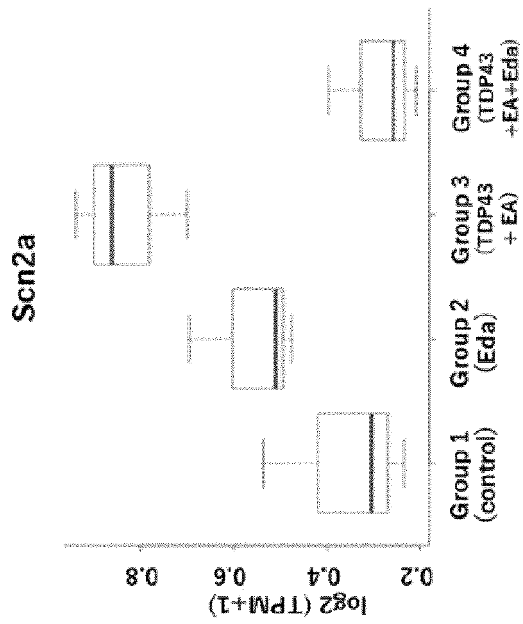


Fig.7(d)

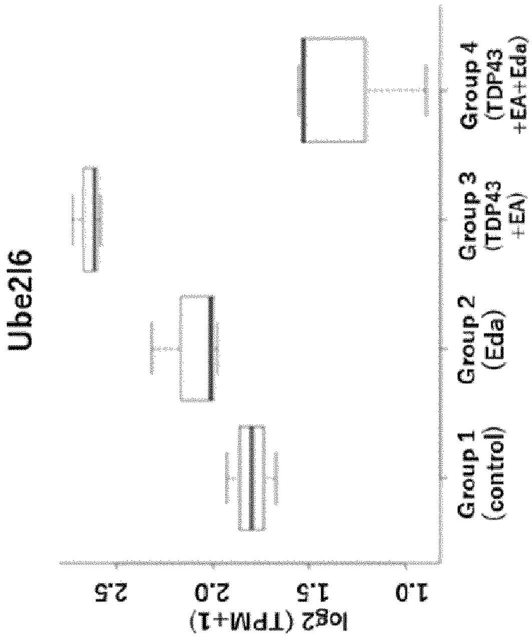

Fig.8(a)

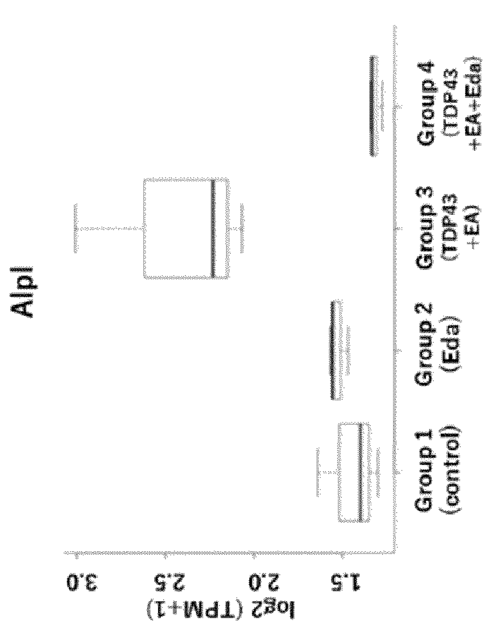


Fig.8(b)

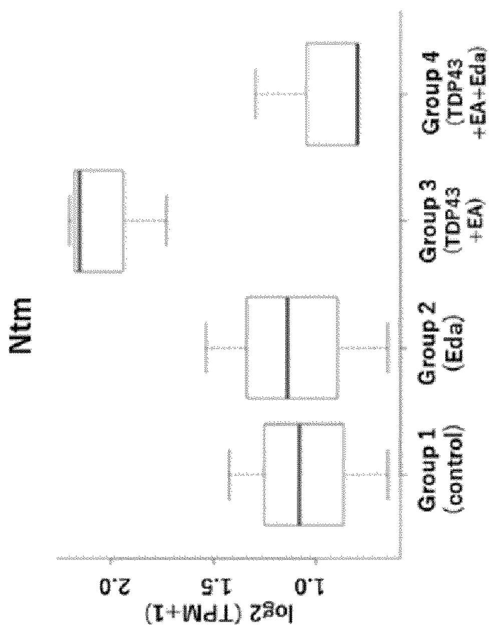


Fig.8(c)

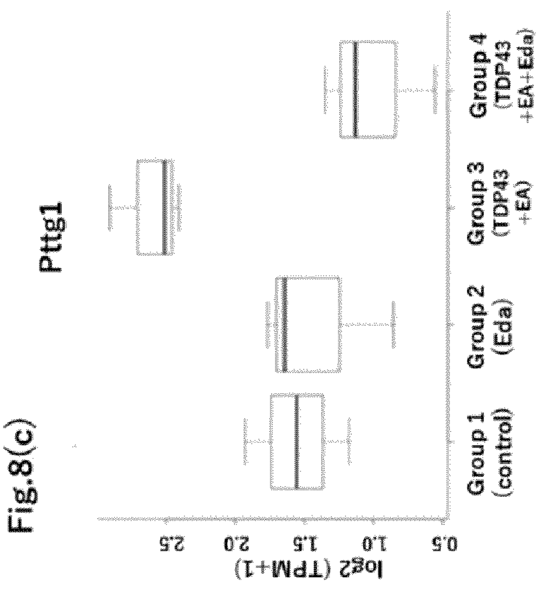


Fig.8(d)

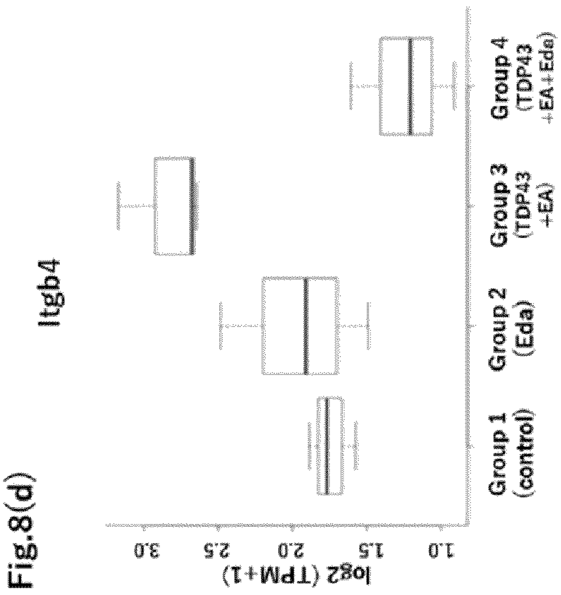

Fig.9(a)

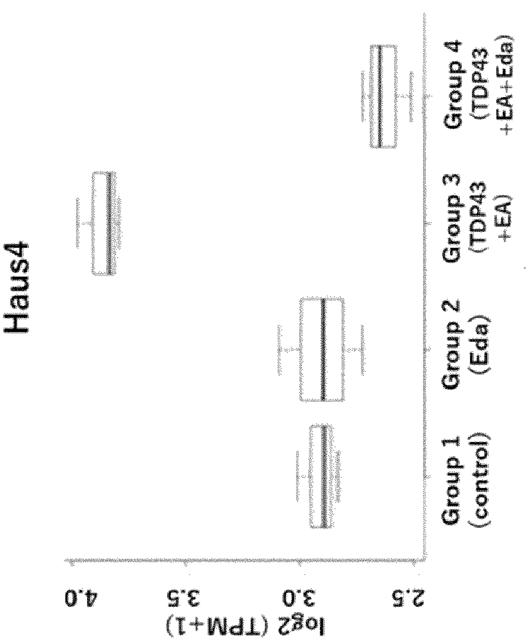


Fig.9(b)

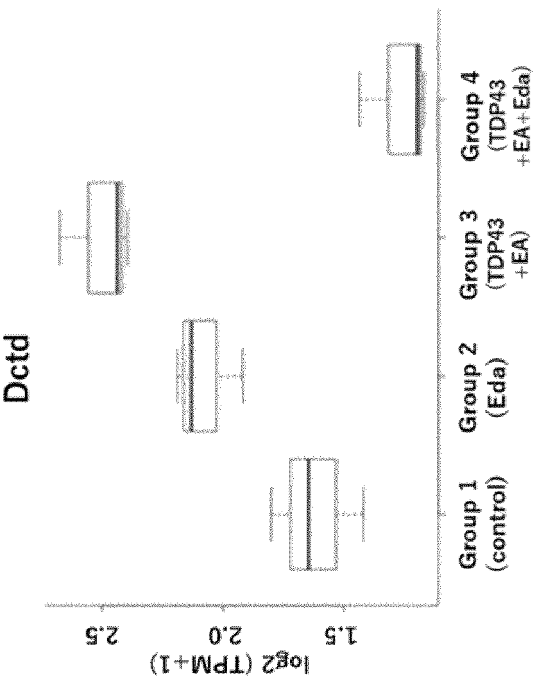


Fig.9(c)

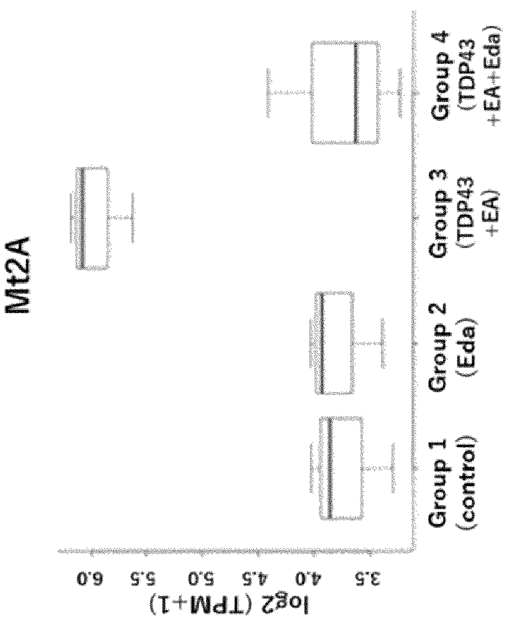


Fig.9(d)

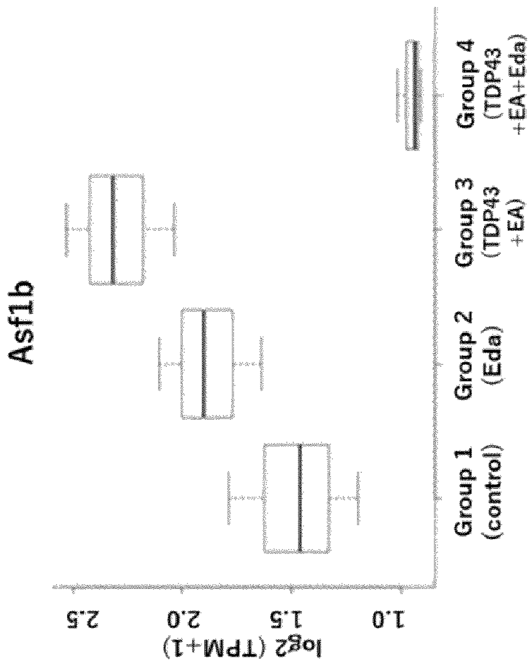

Fig.10(a)

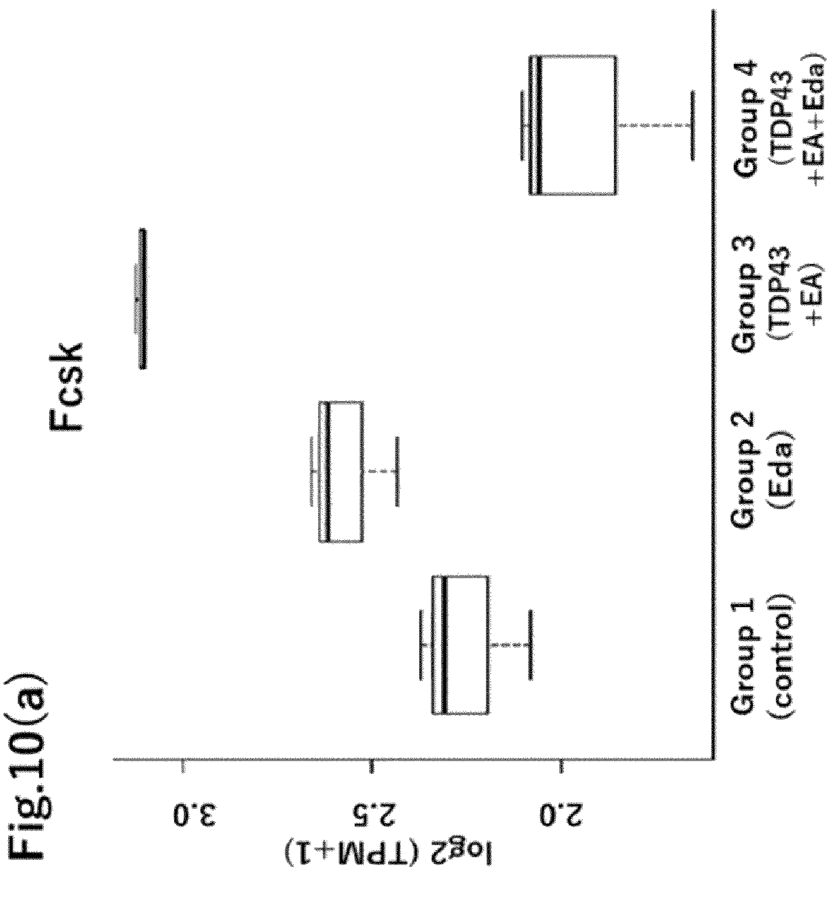


Fig.10(b)

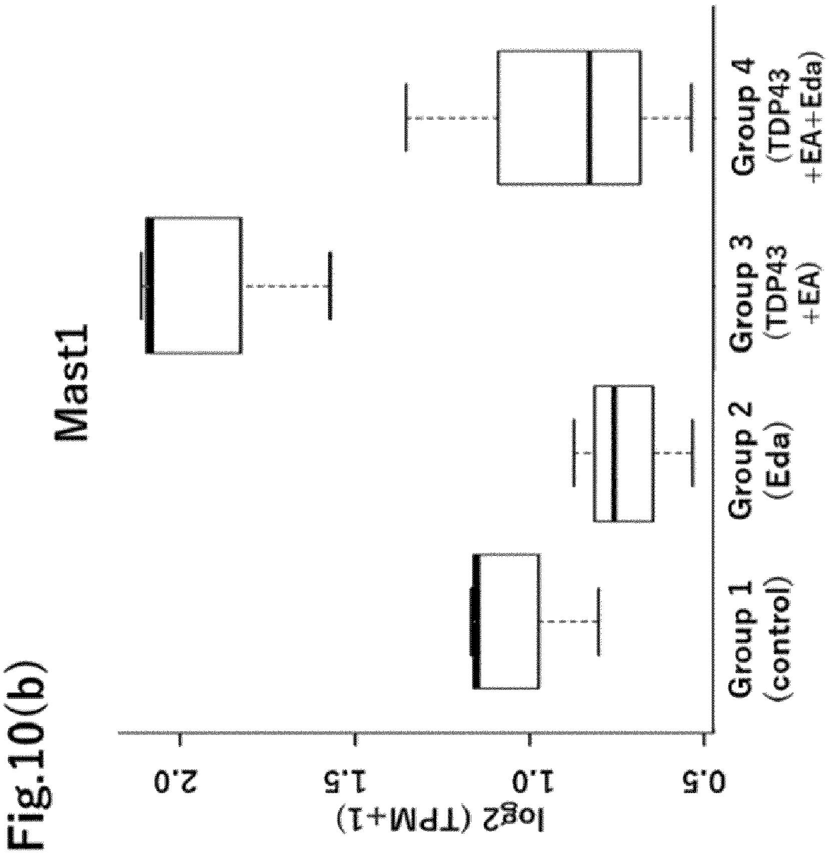

FIG. 11

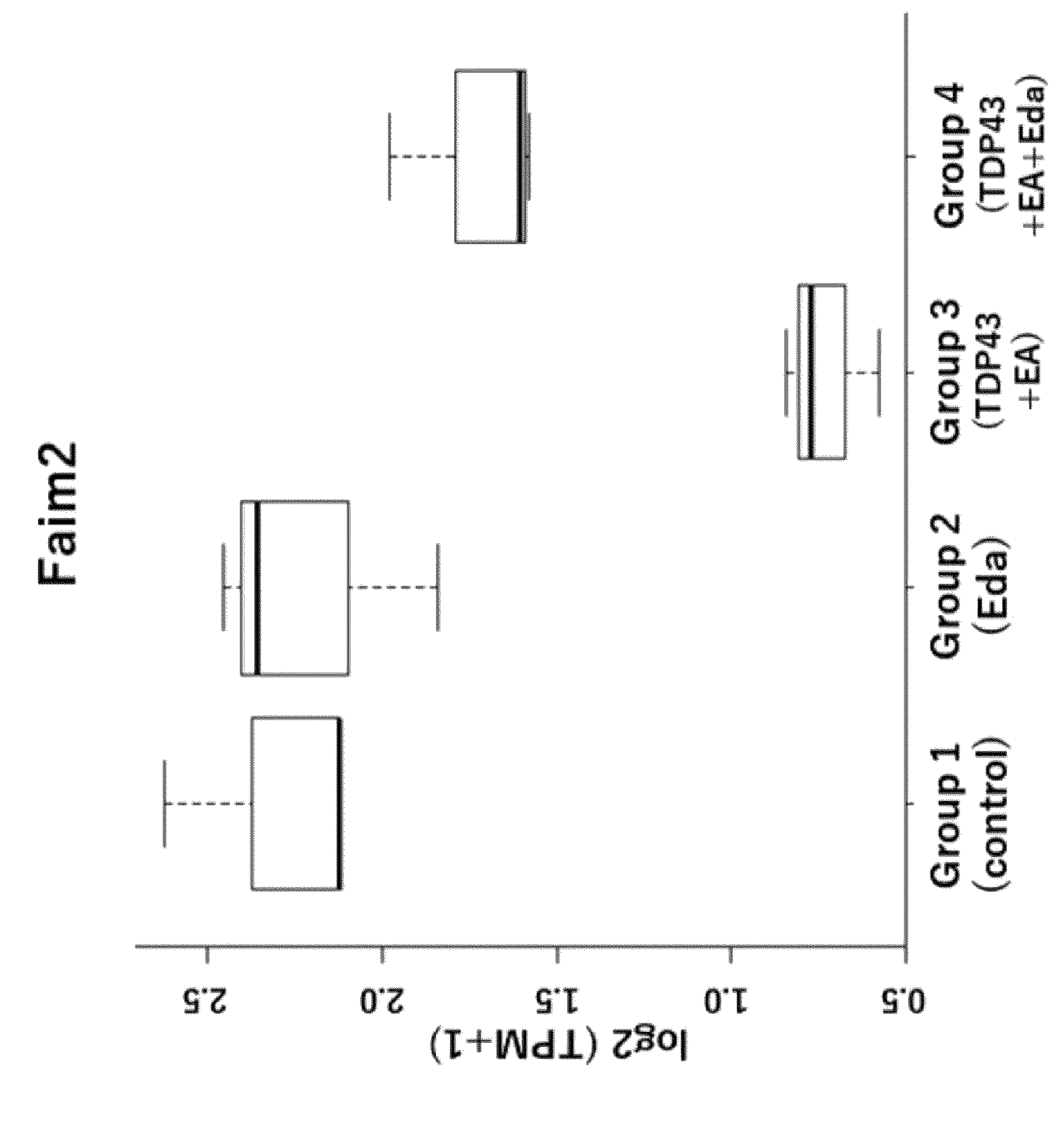
Faim2
log2 (TPM+1)
2.5
2.0
1.5
1.0
0.5
Group 1 (control)
Group 2 (Eda)
Group 3 (TDP43 +EA)
Group 4 (TDP43 +EA+Eda)

FIG. 12

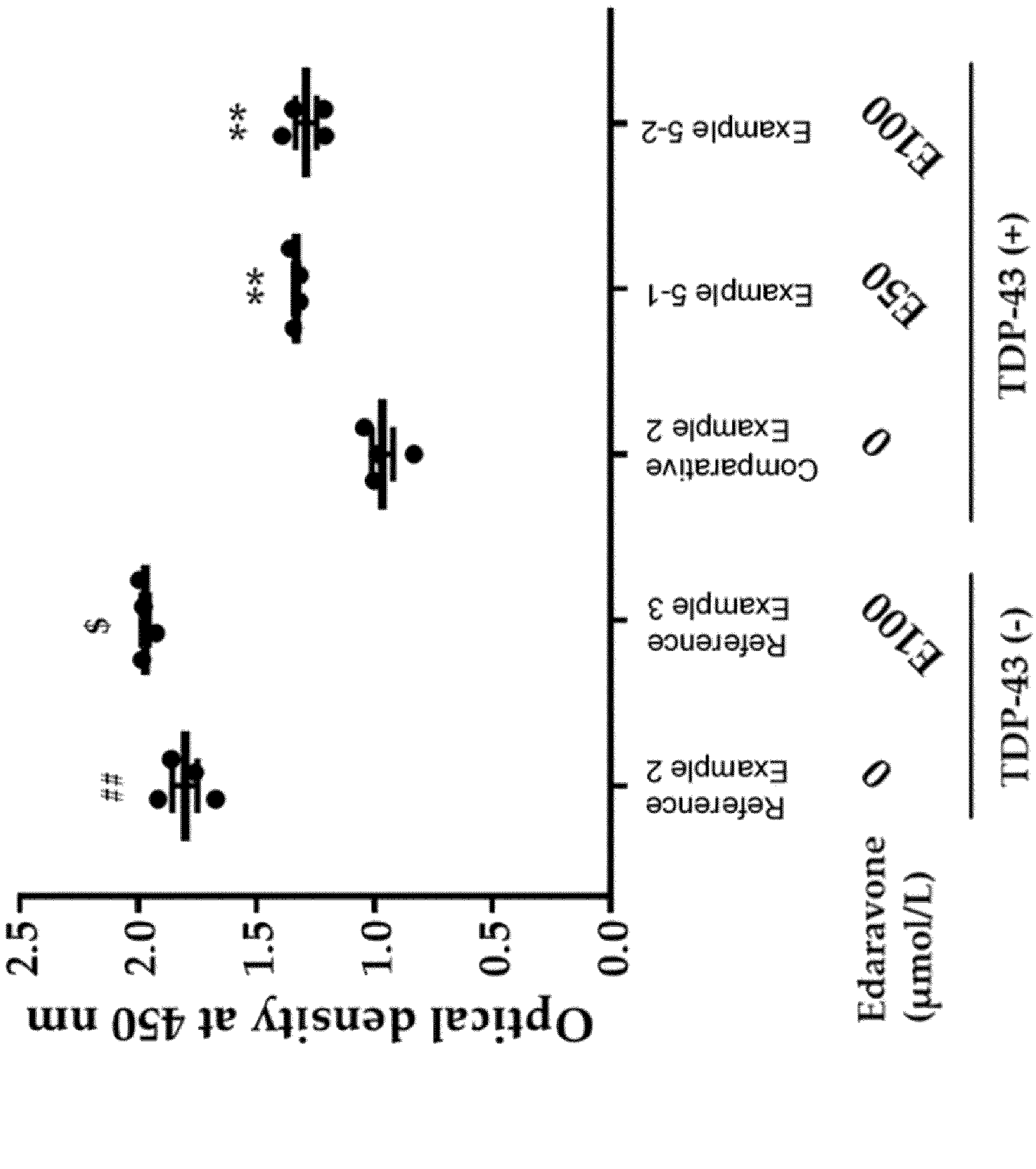
Optical density at 450 nm
2.5
2.0
1.5
1.0
0.5
0.0
##
$
**
**
Reference Example 2
Reference Example 3
Comparative Example 2
Example 5-1
Example 5-2
Edaravone (μmol/L)
0
E100
0
E50
E100
TDP-43 (-)
TDP-43 (+)

FIG. 13

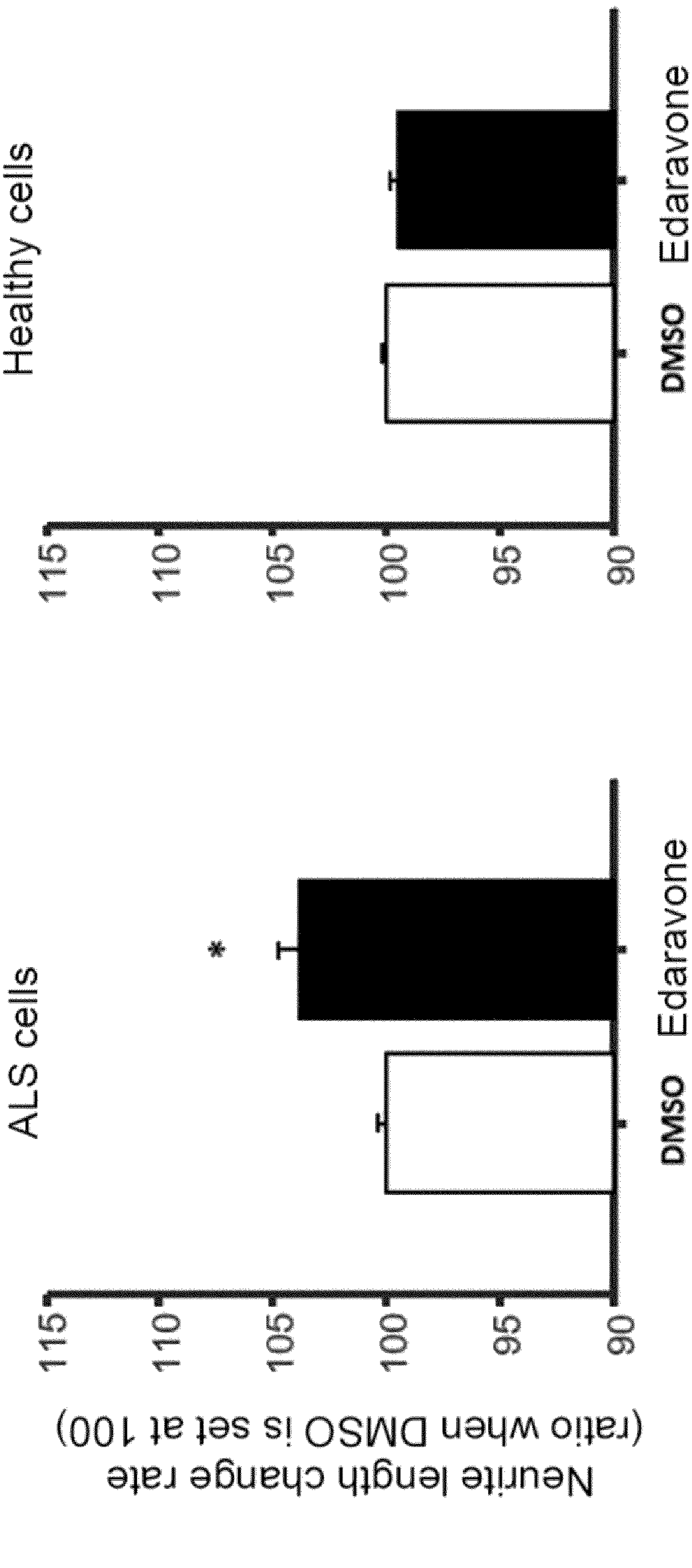

Neurite length change rate
(ratio when DMSO is set at 100)
ALS cells
Healthy cells
115
110
105
100
95
90
*
DMSO
Edaravone

FIG. 14

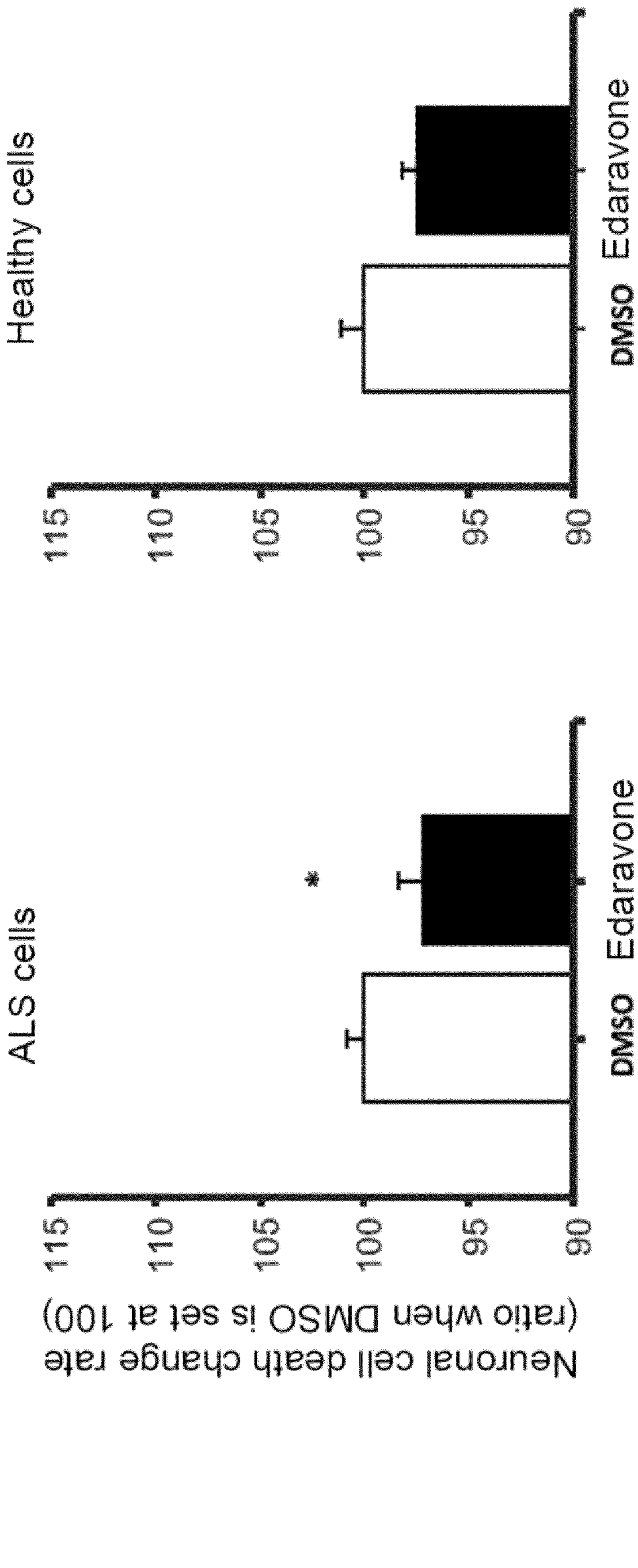
Neuronal cell death change rate
(ratio when DMSO is set at 100)
115
110
105
100
95
90
ALS cells
*
DMSO
Edaravone
Healthy cells
DMSO
Edaravone

FIG. 15

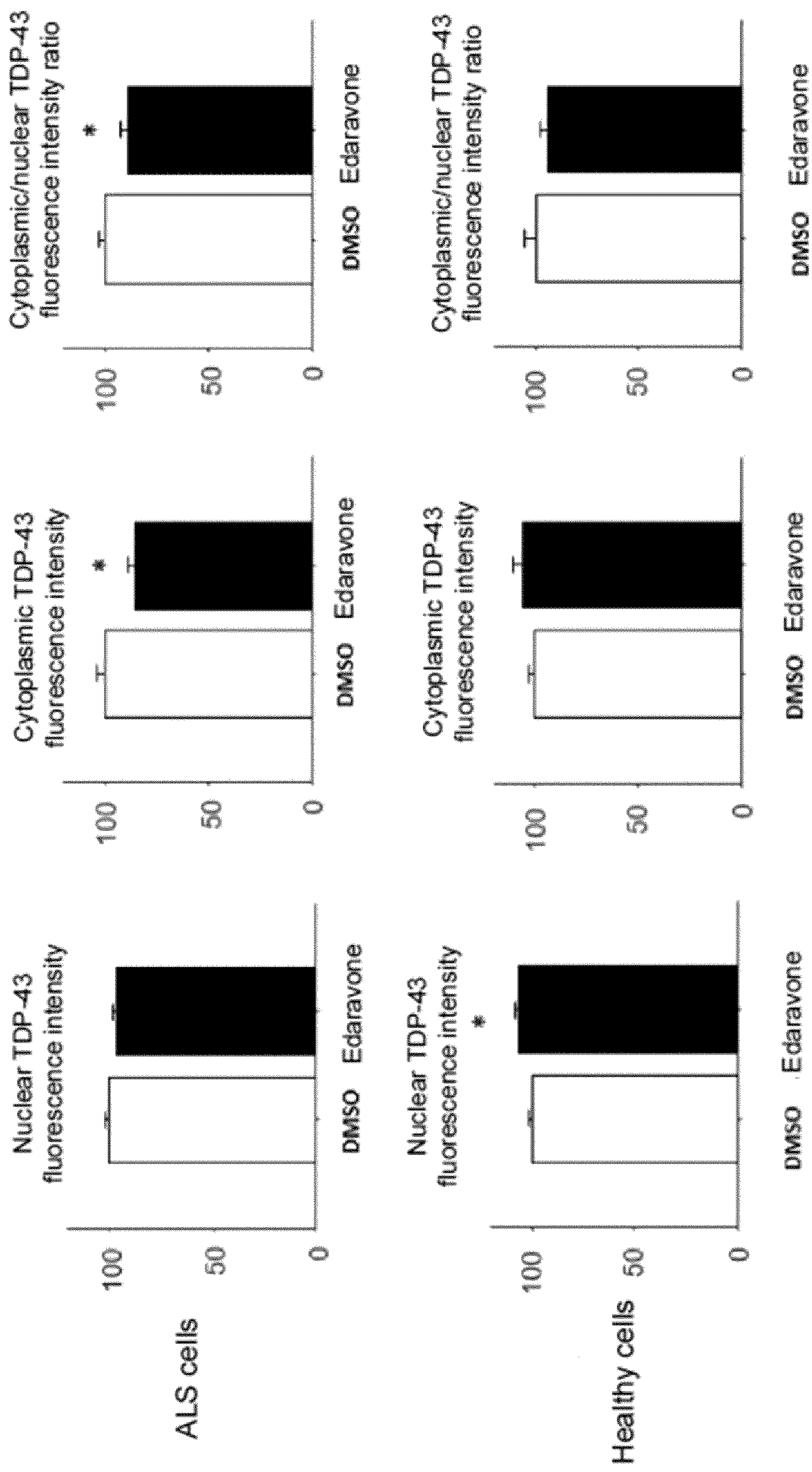
ALS cells
Healthy cells
Nuclear TDP-43 fluorescence intensity
Cytoplasmic TDP-43 fluorescence intensity
Cytoplasmic/nuclear TDP-43 fluorescence intensity ratio
100
50
0
DMSO
Edaravone
*

## INTERNATIONAL SEARCH REPORT

International application No. **PCT/JP2023/024373**

**A. CLASSIFICATION OF SUBJECT MATTER**

***C12Q 1/68***(2018.01)i; ***A61K 31/4152***(2006.01)i; ***A61P 21/00***(2006.01)i; ***A61P 25/00***(2006.01)i; ***A61P 25/28***(2006.01)i; ***C12Q 1/686***(2018.01)i
FI: C12Q1/68; A61K31/4152; A61P21/00; A61P25/00; A61P25/28; C12Q1/686 Z

According to International Patent Classification (IPC) or to both national classification and IPC

**B. FIELDS SEARCHED**

Minimum documentation searched (classification system followed by classification symbols)

C12Q1/68; A61K31/4152; A61P21/00; A61P25/00; A61P25/28; C12Q1/686

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2002/034264 A1 (MITSUBISHI PHARMA CORPORATION) 02 May 2002 (2002-05-02)<br>claims | 1-5 (partially) |
| X | JP 2004-339214 A (MITSUBISHI PHARMA CORPORATION) 02 December 2004 (2004-12-02)<br>paragraphs [0005], [0038], [0040], test example 2, fig. 3-5 | 1-3, 5 (partially) |
| X | JP 5-031523 B2 (MITSUBISHI CHEM IND) 12 May 1993 (1993-05-12)<br>claims | 1-3, 5 (partially) |
| A | JP 2005-531305 A (EVOTEC NEUROSCIENCES GMBH) 20 October 2005 (2005-10-20)<br>entire text, all drawings | 1-5 (partially) |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 September 2023** | **19 September 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2023/024373**

**Box No. III Observations where unity of invention is lacking (Continuation of item 3 of first sheet)**

This International Searching Authority found multiple inventions in this international application, as follows:

Document A: WO 2002/034264 A1 (MITSUBISHI PHARMA CORPORATION) 02 May 2002 (2002-05-02) claims & US6933310 B1 claims & EP 1405637 A1
Document B: JP 2004-339214 A (MITSUBISHI PHARMA CORPORATION) 02 December 2004 (2004-12-02) test example 2, fig. 3-5 (Family: none)
Document C: JP 2005-531305 A (EVOTEC NEUROSCIENCES GMBH) 20 October 2005 (2005-10-20) claims 1, 6 & US2007/0118913 A1 claims 1, 6 & WO2003/104811 A2 & EP1512013 A2

Claims are classified into the following 52 inventions.

(Invention 1) Claims 1-5 (parts relating to KAZALD1)
The invention in claim 1 relates to a composition for changing the expression level of a gene product in a subject, wherein said gene product has KAZALD1, SBK1, SCN2A, UBE2L6, ALPL, NTM, PTTG1, ITGB4, HAUS4, DCTD, MT2A, ASF1B, FCSK, MAST1, and FAIM2 as options.
Hereinafter, whether each option has the same or corresponding special technical feature will be examined.

A composition for changing the expression level of a gene product in a subject, which is an invention that is understood by selecting the first option from among the options in claim 1, wherein the composition contains edaravone and the gene product is KAZALD1, is the invention first set forth in the claims.
The first set forth invention includes the aspect of a composition used to treat or prevent neurodegenerative diseases or ALS as set forth in claims 3 and 4, and lacks novelty in light of document A (in particular, Document A, claims). Alternatively, the first set forth invention merely describes a use of edaravone as a therapeutic agent for ALS, etc. by a newly discovered mechanism of action, and in this regard, it can be said that the first set forth invention lacks novelty in light of document A.
Therefore, the first set forth invention does not have a special technical feature. In addition, this is equally applied to the parts relating to KAZALD1 in the invention in claims 2-5 dependent on claim 1.

Therefore, the parts relating to KAZALD1 in the invention in claims 1-5 are classified as invention 1.

(Invention 2) Claims 1-5 (parts relating to SBK1)
Parts relating to SBK1 in the invention in claim 1 share, with the first set forth invention classified as invention 1, the common technical feature of a composition for changing the expression level of a gene product in a subject, wherein the composition contains edaravone.
However, said technical feature does not make a contribution over the prior art in light of the disclosure of document B (in particular, test example 2, fig. 3-5 in Document B), and thus cannot be said to be a special technical feature. Furthermore, there are no other same or corresponding special technical features between these inventions.

Also, the parts related to SBK1 in the invention in claim 1 are not dependent on invention 1, and are not substantially identical to or similarly closely related to any invention classified as invention 1.
Therefore, the parts relating to SBK1 in the invention in claim 1 cannot be classified as invention 1. In addition, this is equally applied to the parts relating to SBK1 in the invention in claims 2-5.

Therefore, the parts relating to SBK1 in the invention in claims 1-5 are classified as invention 2.

(Inventions 3-15) Claims 1-5 (parts relating to SCN2A-FAIM2)
Similarly to invention 2, the parts relating to the genes of SCN2A, UBE2L6, ..., FAIM2 set forth in claim 1 in the inventions in claims 1-5 are classified as inventions 3-15 in the order set forth, respectively.

(Invention 16) Claims 6 and 10 (parts relating to KAZALD1)
The invention in claim 6 relates to a method for evaluating the response possibility of a subject to edaravone, the method including a step for evaluating whether or not the subject is likely to have responsiveness to edaravone on the basis of changes in the expression level of a gene product due to the exposure of edaravone to the subject, wherein said gene product has KAZALD1, SBK1, SCN2A, UBE2L6, ALPL, NTM, PTTG1, ITGB4, HAUS4, DCTD, MT2A, ASF1B, FCSK, MAST1, and FAIM2 as options.
Hereinafter, whether each option has the same or corresponding special technical feature between inventions 1-15 and each option will be examined.

Form PCT/ISA/210 (continuation of first sheet) (January 2015)

**INTERNATIONAL SEARCH REPORT**

International application No.
**PCT/JP2023/024373**

**Box No. III Observations where unity of invention is lacking (Continuation of item 3 of first sheet)**

Parts relating to KAZALD1 in the invention in claim 6 share, with the first set forth invention classified as invention 1, the common technical feature in that the edaravone is exposed to the subject. In addition, parts relating to KAZALD1 in the invention in claim 6 share the common technical feature of KAZALD1 with the first set forth invention classified as invention 1.

However, the former technical feature does not make a contribution over the prior art in light of the disclosure of document A, and thus cannot be said to be a special technical feature. In addition, the latter technical feature cannot be said to be a special technical feature from the fact that KAZALD1 is a gene known before the priority date of the present application. Therefore, the parts relating to KAZALD1 in the invention in claim 6 cannot be classified as invention 1.

In addition, the parts relating to KAZALD1 in the invention in claim 6 share, with the inventions classified as inventions 2-15, the common technical feature in that the edaravone is exposed to the subject.

However, said technical feature does not make a contribution over the prior art in light of the disclosure of document A, and thus cannot be said to be a special technical feature. Therefore, the parts relating to KAZALD1 in the invention in claim 6 cannot be classified as any of inventions 2-15.

Thus, the parts relating to KAZALD1 in the invention in claims 6 are classified as invention 16. In addition, the parts relating to KAZALD1 in the invention in claim 10 are inventively related to the parts relating to KAZALD1 in the invention in claim 6, and are thus classified as invention 16.

(Invention 17) Claims 6 and 10 (parts relating to SBK1)

The parts relating to SBK1 in the invention in claim 6 cannot be classified as any of inventions 1-15 similarly to invention 16.

In addition, the parts relating to SBK1 in the invention in claim 6 share, with the invention classified as invention 16, the common technical feature of a method for evaluating the response possibility of a subject to edaravone, the method including a step for evaluating whether or not the subject is likely to have responsiveness to edaravone on the basis of changes in the expression level of a gene product due to the exposure of edaravone to the subject.

However, said technical feature does not make a contribution over the prior art in light of the disclosure of document B, and thus cannot be said to be a special technical feature.

Therefore, the parts relating to SBK1 in the invention in claim 6 are classified as invention 17. In addition, the parts relating to SBK1 in the invention in claim 10 are inventively related to the parts relating to SBK in the invention in claim 6, and are thus classified as invention 17.

(Inventions 18-30) Claims 6-10 (parts relating to SCN2A-FAIM2)

Similarly to invention 17, the parts relating to the genes of SCN2A, UBE2L6, ..., FAIM2 set forth in claims 6 and 10 in the inventions in claims 6 and 10 are classified as inventions 18-30 in the order set forth, respectively.

(Invention 31) Claims 7 and 9 (parts relating to KAZALD1)

The invention in claim 7 relates to a method for evaluating the morbidity possibility of a neurodegenerative disease of a subject, the method including a step for evaluating whether or not the subject is likely to have the neurodegenerative disease on the basis of changes in the expression level of a gene product of the subject, wherein the gene product has KAZALD1, SBK1, UBE2L6, NTM, DCTD, ASF1B, and FCSK as options.

Hereinafter, whether each option has the same or corresponding special technical feature between inventions 1-30 and each option will be examined.

Parts relating to KAZALD1 in the invention in claim 7 share, with the first set forth invention classified as invention 1 and the invention classified as invention 16, the common technical feature of KAZALD1.

However, said technical feature cannot be said to be a special technical feature from the fact that KAZALD1 is a gene known before the priority date of the present application. Therefore, the parts relating to SBK1 in the invention in claim 7 cannot be classified as any of inventions 1 and 16.

In addition, the parts relating to KAZALD1 in the invention in claim 7 do not share the common technical feature with the inventions classified as inventions 2-15 and 17-30, and thus cannot be classified as any of inventions 2-15 and 17-30.

Form PCT/ISA/210 (continuation of first sheet) (January 2015)

INTERNATIONAL SEARCH REPORT

International application No.
PCT/JP2023/024373

**Box No. III Observations where unity of invention is lacking (Continuation of item 3 of first sheet)**

Therefore, the parts relating to KAZALD1 in the invention in claim 7 are classified as invention 31. In addition, the parts relating to KAZALD1 in the invention in claim 9 are inventively related to the parts relating to KAZALD1 in the invention in claim 7, and are thus classified as invention 31.

(Invention 32) Claims 7 and 9 (parts relating to SBK1)

The parts relating to SBK1 in the invention in claim 7 cannot be classified as any of inventions 1-30 similarly to invention 31.

In addition, the parts relating to SBK1 in the invention in claim 7 share, with the invention classified as invention 31, the common technical feature of a method for evaluating the morbidity possibility of a neurodegenerative disease of a subject, the method including a step for evaluating whether or not the subject is likely to have the neurodegenerative disease on the basis of changes in the expression level of a gene product of the subject.

However, said technical feature does not make a contribution over the prior art in light of the disclosure of document C (in particular, claim 1), and thus cannot be said to be a special technical feature.

Therefore, the parts relating to SBK1 in the invention in claim 7 cannot be classified as invention 31.

Therefore, the parts relating to SBK1 in the invention in claim 7 are classified as invention 32. In addition, the parts relating to SBK1 in the invention in claim 9 are inventively related to the parts relating to SBK1 in the invention in claim 7, and are thus classified as invention 32.

(Inventions 33-37) Claims 7 and 9 (parts relating to UBE2L6-FCSK)

Similarly to invention 32, the parts relating to the genes of UBE2L6, NTM, ... , FCSK set forth in claims 7 and 9 in the inventions in claims 7 and 9 are classified as inventions 33-37 in the order set forth, respectively.

(Invention 38) Claim 8 (parts relating to KAZALD1)

The invention in claim 8 relates to a method for screening a substance capable of treating or preventing a neurodegenerative disease, the method including a step for selecting a substance to be tested as a substance capable of treating or preventing a neurodegenerative disease on the basis of changes in the expression level of a gene product due to the exposure of the substance to be tested to the subject, wherein said gene product has KAZALD1, SBK1, SCN2A, UBE2L6, ALPL, NTM, PTTG1, ITGB4, HAUS4, DCTD, MT2A, ASF1B, FCSK, MAST1, and FAIM2 as options.

Hereinafter, whether each option has the same or corresponding special technical feature between inventions 1-37 and each option will be examined.

Parts relating to KAZALD1 in the invention in claim 8 share, with the first set forth invention classified as invention 1 and the inventions classified as inventions 16 and 31, the common technical feature of KAZALD1.

However, said technical feature cannot be said to be a special technical feature from the fact that KAZALD1 is a gene known before the priority date of the present application. Therefore, the parts relating to KAZALD1 in the invention in claim 8 cannot be classified as any of inventions 1, 16, and 31.

In addition, the parts relating to KAZALD1 in the invention in claim 8 do not share the common technical feature with the inventions classified as inventions 2-15, 17-30, and 32-37, and thus cannot be classified as any of inventions 2-15, 17-30, and 32-37.

Therefore, the parts relating to KAZALD1 in the invention in claim 8 are classified as invention 38.

(Invention 39) Claim 8 (parts relating to SBK1)

The parts relating to SBK1 in the invention in claim 8 cannot be classified as any of inventions 1-37 similarly to invention 38.

In addition, the parts relating to SBK1 in the invention in claim 8 share, with the invention classified as invention 38, the common technical feature of a method for screening a substance capable of treating or preventing a neurodegenerative disease, the method including a step for selecting a substance to be tested as a substance capable of treating or preventing a neurodegenerative disease on the basis of changes in the expression level of a gene product due to the exposure of the substance to be tested to the subject.

However, said technical feature does not make a contribution over the prior art in light of the disclosure of document C (in particular, claim 6), and thus cannot be said to be a special technical feature.

Therefore, the parts relating to SBK1 in the invention in claim 8 cannot be classified as invention 38.

**INTERNATIONAL SEARCH REPORT**

International application No.
**PCT/JP2023/024373**

**Box No. III Observations where unity of invention is lacking (Continuation of item 3 of first sheet)**

Therefore, the parts relating to SBK1 in the invention in claim 8 are classified as invention 39.

(Inventions 40-52) Claim 8 (parts relating to SCN2A-FAIM2)

Similarly to invention 39, the parts relating to the genes of SCN2A, UBE2L6, ... , FAIM2 set forth in claim 8 in the inventions in claim 8 are classified as inventions 40-52 in the order set forth, respectively.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-5 (parts relating to KAZALD1)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2023/024373**

| Patent document cited in search report | Publication date (day/month/year) | Patent family member(s) | Publication date (day/month/year) |
|---|---|---|---|
| WO 2002/034264 A1 | 02 May 2002 | US 6933310 B1<br>claims<br>EP 1405637 A1 | |
| JP 2004-339214 A | 02 December 2004 | (Family: none) | |
| JP 5-031523 B2 | 12 May 1993 | US 4857542 A<br>claims<br>EP 208874 A1 | |
| JP 2005-531305 A | 20 October 2005 | US 2007/0118913 A1<br>entire text, all drawings<br>EP 1512013 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022106787 A
- JP H531523 B
- US 6933310 B
- EP 208874 A
- WO 2020091036 A


### Non-patent literature cited in the description


- *CHEMICAL ABSTRACTS*, 89-25-8
- **ARAI et al.** *Biochem Biophys Res Commun*, 2006, vol. 351 (3), 602-11
- *Neuropathology*, 2014, vol. 34, 83-98